Europäisches Patentamt

European Patent Office

Office européen des brevets

(19)

(11) EP 0 952 212 A1

(12) EUROPÄISCHE PATENTANMELDUNG

(43) Veröffentlichungstag:
27.10.1999 Patentblatt 1999/43

(51) Int Cl.⁶: C12M 1/36

(21) Anmeldenummer: 99810324.6

(22) Anmeldetag: 19.04.1999

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 20.04.1998 CH 89698

(71) Anmelder: Rohner AG
4133 Pratteln (CH)

(72) Erfinder:
• Péringer, Paul
1028 Préverenges (CH)
• Kneubühler, Werner
4461 Böckten (CH)
• Homann, Hans-Henning
4303 Kaiseraugst (CH)

(74) Vertreter: Zimmermann, Hans, Dr. et al
A. Braun Braun Héritier Eschmann AG
Holbeinstrasse 36-38
4051 Basel (CH)

(54) Verfahren zur aeroben Kultivierung von Mikroorganismen

(57) Ein Verfahren zur aeroben Kultivierung von Mikroorganismen, in welchem
das Substrat durch wiederholte diskontinuierliche Zugabe in jeweils einer Menge zugesetzt wird, die das Wachstum der Mikroorganismen nicht inhibiert und für deren Abbau die im Bioreaktor verfügbare Sauerstoffmenge ausreicht,
die Zufuhr von Sauerstoff während der Substratzugabe unterbrochen ist,
die Zugabe von Substrat jeweils wiederholt wird, wenn das Substrat im wesentlichen verbraucht ist, und
dem Bioreaktor vor der erneuten Zugabe einer Substratmenge Sauerstoff zugeführt wird, wenn die im Bioreaktor vorhandene verfügbare Sauerstoffmenge nicht ausreicht, um die Substratmenge zu abzubauen,
erlaubt eine zuverlässige Vermehrung aerober Mikroorganismen auch unter schwierigen Bedingungen. Es eignet sich insbesondere zur Vermehrung von Mikroorganismen oder Mikroorganismenkonsortien, die auf den Abbau von xenobiotischen Verbindungen und/oder flüchtigen organischen Verbindungen spezialisiert sind, wobei sowohl die Spezialisierung der Mikroorganismen wie auch die Biodiversität vollständig aufrechtzuerhalten werden können.

**Beschreibung**

Die Erfindung betrifft ein Verfahren zur aeroben Kultivierung von Mikroorganismen und insbesondere ein Verfahren zur Massenproduktion von Mikroorganismenkonsortien, die auf den Abbau von flüchtigen organischen Verbindungen und/oder xenobiotischen Verbindungen spezialisiert sind.

Die zurzeit verwendeten industriellen Techniken zur Kultivierung von Mikroorganismen wurden im wesentlichen für die Fortpflanzung von reinen Stämmen entwickelt, die gewisse wirtschaftlich wertvolle Substanzen produzieren können. Diese Methoden können nach ihrer Betriebsweise in diskontinuierliche (batch), halbkontinuierliche (fedbatch) und kontinuierliche (continuous) Verfahren unterteilt werden. Sie werden je nach Fall in Bioreaktoren vom homogenen Typ CSTR (Continuously Stirred Tank Reactor) oder vom heterogenen Typ TFR (Tubular Flow Reactor) aerob oder anaerob durchgeführt. Die Grundlagen, Vorteile und Grenzen dieser Methoden wie auch die geeigneten Bioreaktortypen (Fermenter) und die Kinetik des mikrobiellen Wachstums sind dem Fachmann aus der umfangreichen Literatur bestens bekannt.

Um flüchtige organische Verbindungen (z.B. Lösungsmittel), xenobiotische Verbindungen etc., wie sie z.B. in Industrieemissionen in grossen Menge anfallen können, in Biowäschern, Biofiltern oder Biorieselbettreaktoren biologisch abzubauen, werden vergleichweise grosse Mengen an geeigneten Mikroorganismen benötigt. Die Massenproduktion von Mikroorganismen auf xenobiotischen Verbindungen oder flüchtigen organischen Verbindungen nach bekannten Methoden stösst jedoch auf zahlreiche Schwierigkeiten, wobei insbesondere die folgenden genannt werden können:

- der im allgemeinen recht widerstandsfähige Charakter solcher Schadstoffe, was die Suche nach geeigneten Mikroorganismen und und deren Isolierung, Anpassung und Anreicherung bedingt;
- die meist hydrophobe Natur der Schadstoffe, die ihre Löslichkeit im wässrigen Kulturmedium begrenzt;
- die oft erhöhte Toxizität der Schadstoffe, die die tolerierbare Konzentration im Kulturmedium stark begrenzen kann;
- die Notwendigkeit, gegebenenfalls ein Cosubstrat einsetzen zu müssen, um den Bioabbau und die Produktion leistungsfähiger Mikroorganismen zu ermöglichen;
- die intrazelluläre und/oder extrazelluläre Ansammlung metabolischer Zwischenprodukte, die oft toxischer sind als das Substrat selbst und das mikrobielle Wachstum hemmen können;
- die Desorption (Stripping) von flüchtigen Verbindungen, die nicht nur die Absorptionskinetik des Substrates stört, sondern auch zu Problemen der Sicherheit und des Schutzes des Bedienungspersonals führt.

Die klassischen Batch-Kultivierungsmethoden eignen sich wegen der meist schlechten Löslichkeit der Schadstoffe und aus offensichtlichen ökonomischen Gründen nicht zur Produktion mikrobieller Biomassen auf xenobiotischen und/oder flüchtigen organischen Verbindungen.

Die kontinuierlichen Kultivierungsmethoden sind für die Kultivierung von Mikroorganismen, die auf den Abbau solcher Schadstoffe spezialisiert sind, in der Regel ebenfalls nicht brauchbar, da sie sehr selektiv sind und somit die Biodiversität und die nötigen metabolischen Synergien für gleichzeitigen oder sequentiellen Bioabbau aller Bestandteile eines Substratgemisches nicht aufrechterhalten werden können. Die hohen Verdünnungen, für die die Biomassenproduktivität maximal ist, eliminieren alle Populationen eines Mikroorganismenkonsortiums, deren maximales Wachstum bei diesen Verdünnungen vergleichsweise gering ist. Die Verdünnungen, für die eine akzeptable Biodiversität konserviert werden könnte, ermöglichen hingegen in der Regel keine ökonomische Biomassenproduktivität. Im übrigen hätte die nötige, relativ intensive kontinuierliche Belüftung im Falle flüchtiger Verbindungen einen bedeutenden "Stripping"-Effekt zur Folge, was zu einer instabilen Kultur und den oben angesprochenen Sicherheitsproblemen führen würde.

Die klassischen Fed-Batch-Kultivierungstechniken zeigen im wesentlichen die gleichen Nachteile wie die kontinuierlichen Verfahren. In den Systemen des "Fed-Batch" (Periodic Fed Batch) könnte zwar durch Modulation des Substratzufuhrprofils theoretisch eine Verbesserung erreicht werden. Eine solche Modulation wäre indessen in der Praxis nur mit Hilfe einer extrem kostspieligen instrumentellen Ausrüstung zur Überwachung und rechner-gestützten Steuerung vorstellbar, ohne dass dadurch das Risiko von Messabweichungen infolge der Dynamik des Prozesses völlig ausgeschlossen werden könnte. Zudem könnten auch mit einer solchen Verbesserung weder allfällige Störungen durch metabolische Zwischenprodukte (z.B. solcher, die nur langsam abgebaut werden oder das Mikroorganismenwachstum hemmen) eliminiert, noch die störende Desorption im Falle von flüchtigen Substraten vermieden werden.

Die zukünftige Entwicklung biologischer Behandlungsverfahren und deren Leistungsfähigkeit und Betriebssicherheit wie auch der Aufschwung der biologischen Reinigung von Schadstoffen und industriellen Gasemissionen, die xenobiotische Stoffe und/oder flüchtige organische Verbindungen enthalten, ist deshalb in hohem Masse abhängig von der Entwicklung geeigneter und möglichst ökonomischer Methoden zur Kultivierung entsprechender, auf den Abbau solcher Stoffe spezialisierter Mikroorganismen unter Aufrechterhaltung einer hohen und zeitlich stabilen spezifischen biologischen Aktivität.

Der Erfindung liegt daher die Aufgabe zugrunde, ein Kultivierungsverfahren bereitzustellen, das die Nachteile der vorbekannten Verfahren vermeidet und das sich insbesondere auch zur Vermehrung von Mikroorganismen eignet, die auf den Abbau von xenobiotischen Verbindungen und/oder flüchtigen organischen Verbindungen spezialisiert sind.

Die Aufgabe wird gelöst durch ein Verfahren zur aeroben Kultivierung von Mikroorganismen in einem Bioreaktor unter Verwendung eines wässrigen Kulturmediums und eines als Kohlenstoffquelle dienenden Substrates, wobei das Verfahren dadurch gekennzeichnet ist,

dass dem Bioreaktor Sauerstoff zugeführt und das Substrat durch wiederholte diskontinuierliche Zugabe in jeweils einer Menge zugesetzt wird, die das Wachstum der Mikroorganismen nicht inhibiert und für deren Abbau die im Bioreaktor vorhandene verfügbare Sauerstoffmenge ausreicht,

dass die Zufuhr von Sauerstoff während der Zugabe von Substrat unterbrochen ist,

dass die Zugabe von Substrat jeweils wiederholt wird, wenn die vorherzugeführte Substratmenge im wesentlichen verbraucht ist, und

dass dem Bioreaktor vor der erneuten Zugabe einer Substratmenge Sauerstoff zugeführt wird, wenn die im Bioreaktor vorhandene verfügbare Sauerstoffmenge nicht ausreicht, um die Substratmenge zu abzubauen.

Das erfindungsgemässe Verfahren eignet sich generell zur aeroben Kultivierung grundsätzlich beliebiger, aerober Mikroorganismen, und es erweist sich als gut anpassbare, flexible Kultivierungsmethode, um durch präzise Regelung der Substratzufuhr auch unter schwierigen Bedingungen Inhibition des Zellwachstums zu vermeiden und eine zuverlässige und stabile Produktion zu ermöglichen. Besonders vorteilhaft ist das erfindungsgemässe Verfahren für die Vermehrung von Mikroorganismen oder Mikroorganismenkonsortien, die auf den Abbau von xenobiotischen Verbindungen und/oder flüchtigen organischen Verbindungen spezialisiert sind, wobei sowohl die Spezialisierung der Mikroorganismen wie auch die Biodiversität vollständig aufrechtzuerhalten werden können und zugleich eine vergleichsweise hohe Produktivität erreicht werden kann. Das Verfahren hat sich zudem als äusserst betriebssicher erwiesen, und Risiken von Messabweichungen werden minimiert.

Gemäss dem erfindungsgemässen Verfahren wird das Substrat diskontinuierlich durch wiederholte "Substratpulse" zugesetzt, wobei ein erneuter "Substratpuls" erst erfolgt, wenn die beim vorangehenden "Substratpuls" zugesetzte Substratmenge im wesentlichen verbraucht ist und noch oder - nach vorheriger Sauerstoffzufuhr - wieder eine ausreichende verfügbare Sauerstoffmenge im Bioreaktor vorliegt, um einen erneuten "Substratpuls" abzubauen. Die Substratzufuhr zum Kulturmedium erfolgt somit in "gepulster" Weise, weshalb im Rahmen der vorliegenden Erfindung die einzelnen Substratzugaben auch als "Pulse", die pro Zugabe zugesetzte Substratmenge auch als "Pulsamplitude", der reziproke Wert des zeitlichen Abstandes aufeinanderfolgender Substratzugaben auch als "Pulsfrequenz" und die Methode auch als "Substrat-pulstechnik" bezeichnet werden.

Zur Illustration der Erfindung zeigen

Fig. 1 eine graphische Darstellung typischer Kurvenverläufe der Substrat-, Sauerstoff-, Biomassen- und Metabolitenkonzentration,

Fig. 2 ein Beispiel eines geeigneten Bioreaktors in schematischer Darstellung und

Fig. 3 eine graphische Darstellung der Sauerstoffkonzentration unter Bedingungen, bei denen der Sauerstoffverbrauch zeitweise höher ist als die Sauerstofftransferrate.

Gemäss dem erfindungsgemässen Verfahren kann die Substratzugabe vorzugsweise dadurch erfolgen, dass man flüssiges Substrat in das flüssige Kulturmedium einspritzt. Vorzugsweise wird ein reines Substrat oder ein Gemisch reiner Substrate in flüssiger Form injiziert. Gewünschtenfalls kann auch eine Lösung oder Suspension des Substrates (bzw. der Substrate) in Wasser oder einem ebenfalls abbaubaren organischen Lösungsmittel verwendet werden, wobei aber vorzugsweise konzentrierte Lösungen oder Suspenionen eingesetzt werden, damit das Volumen des Kulturmediums während eines Produktionszyklus annähernd konstant bleibt. Die Substratzugabe kann im allgemeinen relativ rasch erfolgen; in der Regel kann die Zugabe einer Substratmenge, d.h. eines Substratpulses, innert etwa 2 Minuten oder weniger durchgeführt werden, wobei aber längere Zugabezeiten selbstverständlich ebenfalls möglich sind. Wichtig ist hingegen, dass genau kontrollierte Mengen an Substrat zugegeben werden, um die oben genannten Bedingungen einzuhalten. Vorzugsweise kann daher das Substrat mittels eines volumetrischen Injektionssystems eingespritzt werden.

Das erfindungsgemässe Verfahren kann grundsätzlich in einem beliebigen geeigeten Bioreaktor durchgeführt werden, eignet sich aber insbesondere zur Durchführung in einem kontinuierlich gerührten Bioreaktor (Continuously Stirred Tank Reactor). Gewünschtenfalls kann ein geschlossener Bioreaktor verwendet werden, wobei der Ausdruck "geschlossen" in diesem Zusammenhang aber so zu verstehen ist, dass der Reaktorinnenraum allenfalls zwecks Druckausgleich oder Austritt von Schaum z.B. über ein Kapillarrohr oder Luftfilter mit der Umgebung verbunden sein kann und selbstverständlich Sauerstoffzufuhr, Substratzufuhr, Säure- oder Basenzufuhr zur pH-Einstellung und gegebenenfalls Nährstoffzufuhr möglich sein sollen, dass aber mit der Umgebung kein wesentlicher Gasaustausch stattfindet. Die Menge an Kulturmedium kann zweckmässigerweise so gewählt werden, dass im Bioreaktor ein ausreichend grosses Gasvolumen verbleibt, das als Sauerstoffreserve dienen kann. Besonders bewährt haben sich Volumenverhältnisse Gasphase/flüssige Phase von etwa 1:1 bis 2:1. Gewünschtenfalls kann der Reaktor mit einem Ballon versehen werden, um auf diese Weise das nützliche Reaktorvolumen bzw. den Sauerstoffvorrat zu vergrössern und gleichzeitig einen gewissen Druckausgleich zu ermöglichen.

Im erfindungsgemässen Verfahren wird zweckmässigerweise auch der Sauerstoff diskontinuierlich zugeführt, indem die Zufuhr von Sauerstoff zumindest während der Zugabe von Substrat unterbrochen wird. Die erneute Sauerstoffzufuhr kann entweder bereits während des Substratabbaus erfolgen (z.B. wenn Sauerstoff sehr rasch verbraucht wird und/oder Sauerstoff in Form von Luft zugeführt wird) oder vor der nächsten Substratzugabe oder erst nach zwei oder mehreren Substratzugaben, sofern die im Bioreaktor vorhandene verfügbare Sauerstoffmenge dies gestattet. In jedem Fall ist es jedoch bevorzugt, die Sauerstoffzufuhr erst unmittelbar vor der Substratzugabe zu unterbrechen.

Das flüssige Kulturmedium dient als Ernährungsgrundlage für das Wachstum der Mikroorganismen, und die Gasphase bildet das Sauerstoffreservoir für die Atmung und die gleichzeitige Oxidation des Substrates bis auf die Stufe von Kohlendioxid. Als Ernährungsgrundlage eignen sich die üblichen Nährstoffe und Nährmedien. Vorzugsweise wird jedoch ein Nährmedium verwendet, das keine Kohlenstoffquelle enthält, so dass den Mikroorganismen als Kohlenstoffquelle einzig das diskontinuierlich zugesetzte Substrat zur Verfügung steht. Die Konzentration an Vitaminen und Spurenelementen im Kulturmedium wird vorzugsweise in der Weise gewählt, dass sie ein praktisch unbegrenztes mikrobielles Wachstum während des ganzen Produktionszyklus erlaubt, während die Stickstoffquelle vorzugsweise periodisch ergänzt wird. Dadurch erübrigt sich in der Regel eine Ergänzung der Vitamine und Spurenelemente während eines Produktionszyklus.

Die Produktion wird in der Regel fortgesetzt, bis eine Menge an Biomasse erreicht ist, die man in Funktion der Sauerstofftransferkapazität des verwendeten Bioreaktors erhalten kann (d.h. bis die kritische Konzentration an gelöstem Sauerstoff erreicht ist und die Sauerstoffzufuhr ein begrenzender Faktor für die Zellrespiration wird), und dann die Biomasse geerntet. Gewünschtenfalls kann aber die Produktion vorher gestoppt werden, z.B. durch Verwendung einer geringeren Gesamtmenge an Substrat. Ferner kann gewünschtenfalls nur ein Teil der Biomasse geerntet und durch neues Nährmedium ersetzt werden, um einen neuen Produktionszyklus zu starten. Die Dauer eines Produktionszyklus ist variabel und hängt u.a. von der Art der Mikroorganismen und Substrate und von der Anfangskonzentration an Biomasse ab.

Das Substrat (oder Gemisch von Substraten), das als Kohlenstoff- und/oder Energiequelle für das Wachstum der Mikroorganismen dient, wird in diskontinuierlicher Weise, vorzugsweise durch Einspritzen innert relativ kurzer Zeit, zum flüssigen Kulturmedium zugegeben, wo es unter der Wirkung von intensivem mechanischem Rühren rasch verteilt wird. Wie bereits oben erwähnt, wird das Substrat vorzugsweise in flüssiger Form zugegeben, und der Bioreaktor wird während der Dauer der Substratzugabe nicht belüftet, d.h. es wird dem Bioreaktor in dieser Phase kein Sauerstoff zugeführt.

Vorzugsweise kann jede Substratzugabe sofort nach einer Belüftungsperiode (Sauerstoffzufuhr) erfolgen, was die Entfernung von Gasrückständen und die Erneuerung der Sauerstoffreserve erlaubt. Gewünschtenfalls kann aber eine erneute Substratzugabe ohne vorherige Sauerstoffzufuhr erfolgen, wenn der vorhandene Sauerstoffvorrat im Bioreaktor zum Abbau des Substratpulses noch ausreicht. In diesem Fall sollte aber der verfügbare Sauerstoff (z.B. polarographisch mittels einer $pO_2$-Sonde) genau überwacht werden.

Die Sauerstoffzufuhr kann dadurch erfolgen, dass man ein sauerstoffhaltiges Gas, vorzugsweise reinen Sauerstoff, Luft, mit Sauerstoff angereicherte Luft oder ein Sauerstoff/Stickstoff-Gemisch, in den Bioreaktor einleitet, wobei das sauerstoffhaltige Gas beispielsweise direkt in das flüssige Kulturmedium eingeleitet werden kann. Um den Gasaustausch zu beschleunigen und die Gasphase möglichst vollständig zu ersetzen, können die vorhandenen Gase gewünschtenfalls mittels einer Vakuumpumpe entfernt werden.

Um den Sauerstofftransport im Reaktor zu maximieren, kann der im Bioreaktor vorhandene Sauerstoff gewünschtenfalls intern rezirkuliert werden. Dies kann vorzugsweise erfolgen, indem die Gase der Gasphase des Bioreaktors mittels einer Pumpe und einer Verteilereinrichtung durch das flüssige Medium geleitet werden. Die interne Rezirkulation kann vorzugsweise während des Abbaus des Substrates erfolgen und wird während der externen Sauerstoffzufuhr vorzugsweise unterbrochen.

Der Substratpuls kann vorzugsweise mittels eines raschen und präzisen volumetrischen Injektionssystems erfolgen. Im allgemeinen ist es bevorzugt, während eines Produktionszyklus eine konstante Pulsamplitude, d.h. eine konstante Substratmenge pro Zugabe, zu verwenden. Es kann aber gelegentlich erwünscht sein, die Substratmenge während eines Produktionszyklus zu ändern, und gewünschtenfalls können auch variable Substratmengen in Abhängigkeit der Sauerstoffkonzentration, der Konzentration metabolischer Zwischenprodukte etc. zugesetzt werden.

Die optimale Substratmenge und die nötige Sauerstoffmenge können grundsätzlich aus den stöchiometrischen Beziehung für völligen Abbau des Substrates und für Biomassen-bildung berechnet werden. Es ist aber in jedem Fall zu empfehlen, Vorversuche durchzuführen, um diese Werte zu überprüfen, um unerwartete Einflüsse durch metabolische Zwischenprodukte zu berücksichtigen und um die Zeitintervalle zwischen aufeinanderfolgenden Substratzugaben bzw. Belüftungen sowie für die Belüftungsdauer und die Abbauphase zu optimieren.

Die Pulsfrequenz bzw. das Zeitintervall zwischen aufeinanderfolgenden Substratzugaben ist vor allem abhängig von der Substratabbaugeschwindigkeit und kann entweder konstant gehalten oder in Abhängigkeit des Verfahrensverlaufs variiert werden.

Bei prozessgekoppelt-offener Durchführung (open loop) des Verfahrens wird eine konstante Pulsfrequenz und eine konstante Belüftungsfrequenz angewendet, d.h. die erneute Belüftung und die erneute Substratinjektion erfolgen jeweils nach konstanten Zeitintervallen, die aufgrund der kinetischen Eigenschaften der Mikroorganismen im voraus definiert und vorzugsweise in Vorversuchen getestet werden. Diese Verfahrensweise ist im allgemeinen robust, funktions-sicher und mit Hilfe eines programmierbaren Automaten einfach umzusetzen, erlaubt aber meist nicht, eine maximale Biomassenproduktivität über den gesamten Batch zu erreichen.

Bei prozessgekoppelt-geschlossener Durchführung (closed loop) des Verfahrens sind die Pulsfrequenzen und die Wiederbelüftungsfrequenzen (reziproker Wert des Intervalles zwischen zwei aufeinanderfolgenden Starts der Sauerstoffzufuhr) variabel und im allgemeinen bis zum Ende eines Produktionszyklus steigend. Die Frequenzen bzw. die entsprechenden Zeitintervalle sind insbesondere abhängig vom Ent-wicklungsprofil der Konzentration an gelöstem Sauerstoff im flüssigen Kulturmedium, die kontinuierlich, z.B. mit Hilfe einer polarographischen Sonde und gegebenenfalls zusammen mit weiteren on-line oder off-line erfassten Parametern (z.B. die Messung der Absorption bei 255 nm), gemessen wird.

In beiden Durchführungsarten ist das erfindungsgemässe Verfahren vorzugsweise gekennzeichnet durch eine Wiederholung der Sequenz von Verfahrensschritten, umfassend die Zufuhr von Sauerstoff, Unterbrechung der Sauerstoffzufuhr und ein- oder mehrmalige Zugabe von Substrat. Falls die Gase zur Verbesserung des Sauerstofftransportes intern rezirkuliert werden, ergeben sich als weitere bevorzugte Verfahrensschritte: Inbetriebnahme der Rezirkulation nach Unterbrechung der Sauerstoffzufuhr und Unterbrechung der Rezirkulation vor erneuter Sauerstoffzufuhr, wenn die Rezirkulation nur während der Sauerstoffzufuhr unterbrochen wird, bzw. Inbetriebnahme der Rezirkulation nach Zugabe von Substrat und Unterbrechung der Rezirkulation vor Sauerstoffzufuhr oder erneuter Zugabe von Substrat.

Eine typische Abfolge von Massnahmen und Verfahrensschritten lässt sich z.B. im Falle wiederholter Produktion nach einer prozessgekoppelt-offenen Verfahrensvariante wie folgt veranschaulichen:

1. Berechnung der Pulsamplituden, der Pulsfrequenzen, der Dauer der Wiederbelüftungsphasen des Mediums und der kritischen Konzentration für den gelösten Sauerstoff;
2. Wahl einer ausreichenden Menge eines geeigneten Nährmediums;
3. Stabilisierung und vorzugsweise automatische Regulierung der Temperatur und des pH auf ihren Sollwerten unter nominal konstanter Belüftung und Inokulierung des Bioreaktors mit dem Mikroorganismus bzw. den Mikroorganismen;
4. gegebenenfalls Initialisierung des programmierbaren Automaten;
5. Belüftung des Mediums während des festgelegten Zeitintervalls;
6. Unterbrechen der Belüftung und Schliessen des Gasaustrittsventils (und gegebenenfalls Öffnen eines Druckausgleichsventils);
7. Injektion einer festgelegten Substratmenge;
8. gewünschtenfalls Inbetriebnahme einer gegebenenfalls vorhandenen internen Gasrezirkulation;
9. Unterbruch der gegebenenfalls vorhandenen internen Gasrezirkulation nach einem festgelegten Zeitintervall;
10. Öffnen des Gasaustrittsventils und Wiederbelüftung (Sauerstoffzufuhr) des Mediums während der festgelegten Zeitdauer;
11. mehrfache Wiederholung der Schritte 5-9;
12. Anhalten des Zyklus am Ende von Schritt 8;
13. Ernten der Biomasse durch Entnahme eines Teils des erschöpften Mediums;
14. Ersetzen des erschöpften Mediums durch ein entsprechendes Volumen an neuem Medium;
15. Initiierung eines neuen Produktionszyklus.

Schritte 1-4 erfolgen vorzugsweise von Hand; Schritte 5-15 können vorzugsweise automatisch durchgeführt werden, falls eine entsprechende automatische Steuerung verwendet wird. Vor der Produktion grösserer Mengen an Biomasse ist es in der Regel zu empfehlen, die berechneten Zeiten und Mengen in Vorversuchen zu testen und notfalls zu optimieren.

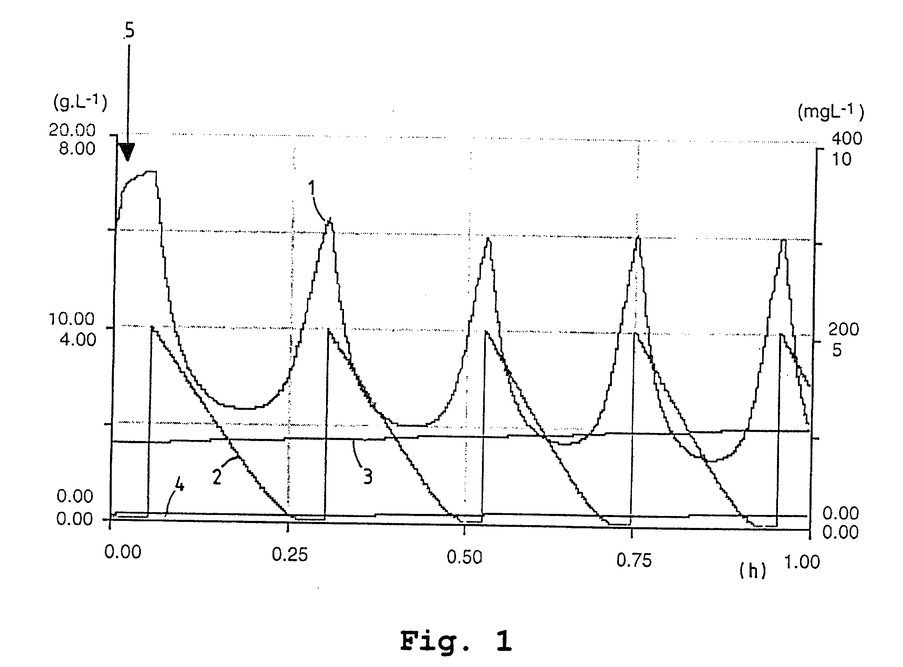

Fig. 1 zeigt in graphischer Darstellung typische Kurvenverläufe für die Konzentration an gelöstem Sauerstoff 1 (Skala 0-8 g/l), die Substratkonzentration 2 (Skala 0-400 mg/l), die Konzentration an Biomasse 3 (Skala 0-20 g/l) und die Konzentration an Metaboliten 4 (Skala 0-10 mg/l) bei prozessgekoppelt-offener Durchführung des Verfahrens. Eine Belüftung erfolgte im gezeigten Ausschnitt nur an der mit Pfeil 5 markierten Stelle. Die Maxima der Substratkonzentrationskurve entsprechen der "Pulsamplitude" und der zeitliche Abstand zwischen zwei aufeinanderfolgenden Substratzugaben dem reziproken Wert der "Pulsfrequenz".

Weitere Verfahrensparameter, wie pH-Wert, Temperatur, Sauerstoffkonzentration und dergleichen, sind vom verwendeten Mikroorganismus und Substrat abhängig und können von Fall zu Fall leicht optimiert werden. In den meisten Fällen hat sich jedoch ein pH-Wert von etwa 7, eine Temperatur von etwa 30°C und eine hohe Sauerstoffkonzentration (vollständige oder weitgehende Sauerstoffsättigung bei der Belüftung) bewährt. Falls zur Zufuhr von Sauerstoff Luft verwendet wird, sind meist etwas längere Belüftungsphasen und/oder eine interne Gasrezirkulation zu empfehlen, insbesondere wenn der Sauerstoffverbrauch des Systems hoch ist. Ferner sollte darauf geachtet werden, dass die Konzentration freigesetzter Chloridionen im Kulturmedium nicht zu hohe Werte erreicht und beispielsweise nicht über etwa 0,3 M steigt.

Das erfindungsgemässe Verfahren eignet sich grundsätzlich zur Kultivierung beliebiger aerober Mikroorganismen und insbesondere zur Kultivierung von Mikroorganismen, die auf den Abbau flüchtiger organischer Verbindungen (insbesondere Lösungsmittel) und/oder xenobiotischer Verbindungen spezialisiert sind, wie sie beispielsweise in Gasemissionen der Industrie (z.B. Chemiebetrieben) anfallen. Ein besonders bevorzugter Aspekt betrifft die Kultivierung von Mikroorganismen, die auf den Abbau chlorierter bzw. nicht chlorierter, aromatischer oder aliphatischer Lösungsmittel, insbesondere nicht wassermischbarer (d.h. nicht im beliebigen Verhältnis mit Wasser mischbarer) Lösungsmittel, wie Monochlorbenzol, Dichlorbenzol, Trichlorbenzol, Dichlorethylen, Trichlorethylen, Dichlormethan, Epichlorhydrin, Benzol, Toluol, Xylol, Ethylbenzol, Styrol und dergleichen, spezialisiert sind.

Ferner eignet sich das erfindungsgemässe Verfahren nicht nur zur Kultivierung eines einzelnen Mikroorganismus, sondern vorzugsweise auch zur Kultivierung von gemischten Mikroorganismenpopulationen, sogenannten Mikroorganismenkonsortien, insbesondere solchen, die auf den Abbau mehrerer oder einer ganzen Klasse flüchtiger organischer Verbindungen und/oder xenobiotischer Verbindungen spezialisiert sind. Werden nämlich den Mikroorganismus oder Mikroorganismenkonsortien als Substrate nur die Verbindungen bzw. Gemische von Verbindungen, auf deren Abbau sie spezialisiert sind, als einzige Kohlenstoffquelle angeboten, so wird nicht nur eine hohe Bioausbeute erzielt, sondern gewährleistet, dass die Spezialisierung und die Biodiversität (und somit die metabolischen Synergien) konserviert werden, da das Wachstum mutierter ungeeigneter Mikroorganismen wirksam verhindert wird.

Das erfindungsgemässe Verfahren eignet sich somit insbesondere auch vorzüglich zur Produktion spezialisierter Mikroorganismen und Mikroorganismenkonsortien, die in Biowäschern, Biofilter oder Biorieselbettreaktoren zum Bioabbau industrieller Gasemissionen oder auch zur Behandlung kontaminierter Böden und Grundwässer Verwendung finden.

Ein besonders bevorzugter Aspekt der vorliegenden Erfindung betrifft daher die Vermehrung von Mikroorganismen, die auf den Abbau chlorierter aromatischer oder aliphatischer Lösungsmittel spezialisiert sind, wobei als Substrat ein chloriertes aromatisches oder aliphatisches Lösungsmittel, auf dessen Abbau die Mikroorganismen spezialisiert sind, oder ein Gemisch solcher Lösungsmittel verwendet wird. Ein weiterer, besonders bevorzugter Aspekt betrifft ferner die Vermehrung von Mikroorganismen, die auf den Abbau nicht chlorierter aromatischer oder aliphatischer Lösungsmittel spezialisiert sind, wobei als Substrat ein nicht chloriertes aromatisches oder aliphatisches Lösungsmittel, auf dessen Abbau die Mikroorganismen spezialisiert sind, oder ein Gemisch solcher Lösungsmittel verwendet wird.

Auf den Abbau chlorierter oder nicht chlorierter organischer Verbindungen spezialisierte Mikroorganismen, wie beispielsweise Mikroorganismen der Gattungen Pseudomonas, Nocardia, Alcaligenes, Arthrobacter, Xanthobacter, Comamonas, Rhodococcus, Geobacter, Geotrichum, Azoarcus, Spingomonas, Aeromonas, Corynebacterium und Burkholderia, sind dem Fachman bekannt und in der Umwelt weit verbreitet und/oder bei anerkannten Hinterlegungsstellen, wie American Type Culture Collection, Rockville, Maryland, USA (ATCC), DSM-Deutsche Sammlung von Mikroorganismen und Zellkulturen, Braunschweig, Deutschland (DSM) oder Agricultural Research Service Culture Collection, Peoria, Illinois, USA (NRRL), hinterlegt. Gemischte Mikroorganismenpopulationen können beispielsweise erhalten werden durch Kombination von reinen Stämmen auf den Abbau einzelner Verbindungen spezialisierter, nicht pathogener, in öffentlichen oder privaten Sammlungen verfügbarer Bakterien (z.B. Pseudomonas putida 3226, 6537, Pseudomonas GJ1, GJ3, GJ8, GJ 30, GJ 31, GJ 40, Pseudomonas stutzeri, Nocardia GJ 100, Alcaligenes A 175 etc.) oder durch Kombination von reinen Stämmen von Bakterien, die aus einem mit der betreffenden Verbindung kontaminierten Milieu isoliert wurden und einer geeigneten nicht pathogenen Gattung (z.B. Pseudomonas spp, Alcaligenes spp, Arthrobacter spp, Nocardia spp, Xanthobacter spp, Comamonas spp etc.) angehören.

Für den Abbau chlorierter organischer Verbindungen eignen sich beispielsweise Stämme wie: Pseudomonas putida ATCC 53645, Pseudomonas sp. DSM 6838, Pseudomonas sp. DSM 7116, Pseudomonas sp. NRRL-B-12538, Pseudomonas sp. NRRL-B-12539 und dergleichen. Als Beispiele geeigneter Mikroorganismen für den Abbau nicht chlorierter Verbindungen wie Benzol, Toluol, Xylol, Ethylbenzol etc. können folgende Stämme genannt werden: Pseudomonas putida DSM 548, Pseudomonas putida DSM 6414, Pseudomonas putida DSM 3226, Pseudomonas putida DSM 6413, Pseudomonas putida ATCC 700007, Pseudomonas putida ATCC 700008, Pseudomonas putida ATCC 700007, Pseudomonas putida ATCC 39213, Pseudomonas testosteroni DSM 6577, Pseudomonas fluorescens DSM 6607, Pseudomonas fluorescens DSM 6608, Pseudomonas fluorescens DSM 6609, Pseudomonas stutzeri DSM 6538, Pseudomonas sp. DSM 6611, Pseudomonas sp. DSM 6708, Pseudomonas sp. DSM 6818, Pseudomonas sp. DSM 6838, Pseudomonas sp. DSM 7116, Pseudomonas sp. DSM 6537, Pseudomonas sp. ATCC 51283, Alcaligenes sp. DSM 6610, Comamonas testosteroni ATCC 27911, Xanthobacter sp. DSM 6696, Geobacter metallireducens DSM 7210, Azoarcus tolulyticus ATCC 51758, Sphingomonas sp. DSM 6900, Aeromonas spp. ATCC 55642, Aeromonas spp. ATCC 55641, Corynebacterium sp. ATCC 55643, Zoogloea sp. ATCC 55649, Rhodococcus zopfii ATCC 51349 Rhodococcus zopfii ATCC 51349. Ferner sind auch Mikroorganismen bekannt, die sich speziell für den Abbau von organischen Schwefelverbindungen eignen; beispielsweise werden in EP-A-0 669 155 Mikroorganismen der Gattung Hyphomicrobium für diesen Zweck beschrieben. Weiterhin sind auch für den Abbau wasserlöslicher organischer Verbindungen geeignete Mikroorganismen in grosser Zahl bekannt und dem Fachmann bestens geläufig.

Gemäss einer bevorzugten Methode werden jedoch in der Umwelt vorkommende Mikroorganismen, vorzugsweise gemischte Mikroorganismenpopulationen, verwendet, die aus einem mit der abzubauenden Verbindung bzw. den abzubauenden Verbindungen kontaminierten Milieu, beispielsweise einem Industriegelände oder einer Abwasserreinigungsanlage, gewonnen werden. Die Isolierung und anschliessende Auswahl, Adaptierung, Anreicherung und Kultivierung der Mikroorganismen kann nach klassischen mikrobiologischen Methoden erfolgen. Vorzugsweise wird die Kultivierung auf einem „Minimalmilieu" (DSM 457) durchgeführt, das neben den abzubauenden Verbindungen keine weitere Kohlenstoffquelle enthält. Gewünschtenfalls kann die Kultivierung auch in Verbindung mit einer exogenen Quelle wie Methanol oder auf einem Nährstoffmedium von Typ Plate Count Agar (PCA) oder Nutrient Broth (NB) durchgeführt werden.

Solche aus einem kontaminierten Milieu gewonnenen Mikroorganismenkonsortien zeichnen sich im allgemeinen durch hohe Wachstumsraten, hohe Widerstandsfähigkeit gegen erhöhte Schadstoffkonzentrationen, Robustheit und leichte Konservierbarkeit aus. Vorzugsweise werden jeweils mehrere derartige Mikroorganismenkonsortien gewonnen und dann gemischt und weiter angereichert oder aufgrund der biologischen Parameter (wie maximale Wachstumsrate, Wachstumsausbeute, Atmungs- und Bioabbaugeschwindigkeit, Grad der Bildung ausgeschiedener Zwischenprodukte, tolerierte Maximalkonzentration an abzubauenden Verbindungen und Sättigungskonstanten) das beste ausgewählt.

Wie in den Beispielen beschrieben wird, wurden auf diese Weise aus Proben der Abwasserreinigungsanlage der Gemeinde Schweizerhalle (Schweiz) ein auf den Abbau nicht chlorierter aromatischer Verbindungen spezialisiertes Konsortium, umfassend Burkholderia sp., Pseudomonas putida, Pseudomonas mucidolens, Pseudomonas cepecia, Pseudomonas flava, Stenotropphomonas sp. und Comamonas sp., und ein auf den Abbau chlorierter aromatischer Verbindungen spezialisiertes Konsortium, umfassend Pseudomonas mucidolens, Pseudomonas putida Biotyp B, Pseudomonas fluorescens Biotyp C, Comamonas acidovorens, Arthrobacter uratoxydans, Rhodococcus erythropili und Bacillus cereus, gewonnen.

Die Erfindung wird durch die folgenden Beispiele weiter veranschaulicht. Sofern in den Beispielen nicht etwas Abweichendes ausgesagt wird, wurde als Kulturmedium jeweils das in Beispiel 2 definierte Kulturmedium A ohne Kohlenstoffquelle verwendet. TEX bezeichnet ein Gemisch aus 90 Vol.-% Toluol, 2,5 Vol.-% Ethylbenzol, 6,2 Vol.-% m- und p-Xylol und 1,3 Vol.-% o-Xylol.

Beispiel 1

Produktion eines spezialisierten Mikroorganismenkonsortiums auf einem Gemisch von Monochlorbenzol und o-Dichlorbenzol

a) Auswahl leistungsfähiger Mikroorganismenkonsortien

In der Abwasserreinigungsanlage der Gemeinde Schweizerhalle (Schweiz) wurden mehrere Proben aus den kontaminierten Belebtschlämmen und den Abgas-Biofiltrationseinheiten entnommen und in Schüttelkolben auf ihre Fähigkeit zum Bioabbau von Monochlorbenzol und o-Dichlorbenzol getestet. Die Durchführung der Tests erfolgte in 500 ml-Erlenmeyerkolben mit einem Glas-Seitenarm, um das Wachstum durch Messung der Extinktion bei 650 nm zu verfolgen, sowie einem Mininert-Ventil und einem Teflon-Butyl-Septum, um mit einer gasdichten 250 ml-Spritze (mit Ventilen) Gasproben zur GC-Analyse (Varian GB 3400 Cx. Column J&W Scientific DB-624) entnehmen zu können. Die Kolben wurden mit 100 ml Minimalkulturmedium (pH 7,0), enthaltend Ammoniumsulfat als Stickstoffquelle, gefüllt, mit 1 g Probe angeimpft und dann auf einer Umlaufschüttelmaschine bei 30°C und 110 UpM inkubiert. Durch Einleiten von reinem Sauerstoff in die Kolben erreichte die Anfangskonzentration an gelöstem Sauerstoff 35 mg/l (7 mg/l im Falle der Sättigung mit Luft) und der Sauerstoffgehalt der Gasphase war nahezu 100%. Nach Sättigung der Kolben mit reinem Sauerstoff wurde durch das Mininert-Ventil und das Septum Monochlorbenzol, o-Dichlorbenzol oder ein Gemisch der beiden als einzige Kohlenstoffquelle in die Kolben injiziert. Die eingespritzte Substratmenge wurde in einem Bereich von null bis zur Sättigungskonzentration im Kulturmedium variiert.

Um sicherzugehen, dass genügend Sauerstoff und Stickstoff vorliegt, wurde der Sauerstoff- und Stickstoffbedarf vorab aufgrund der stöchiometrischen Gleichungen für die vollständige Oxidation des Substrates und für die Biomassenbildung abgeschätzt unter der Annahme einer Biomassenausbeute von 0,5 g Trockengewicht pro 1 g Substrat. Beispielsweise erfolgte die Berechnung für Monochlorbenzol anhand der Gleichungen:

Oxidation:

$$C_6H_5Cl + 7 O_2 \rightarrow 6 CO_2 + 2 H_2O + 1 HCl$$

Biomassensynthese:

$$C_6H_5Cl + {}^6/_5 NH_3 + 1 O_2 + {}^2/_5 H_2O \rightarrow {}^6/_5 C_5H_7NO_2 + 1 HCl$$

Oxidation und Biomassensynthese:

$$C_6H_5Cl + 0{,}448 NH_3 + 4{,}758 O_2 \rightarrow 0{,}448 C_5H_7NO_2 + 3{,}759 CO_2 + 1{,}074 H_2O + 1 HCl$$

Drei der getesteten Mikroorganismenproben erwiesen sich als fähig, Monochlorbenzol und Dichlorbenzol als Einzelverbindungen abzubauen. Die vom Biofilter stammende Probe hatte die breiteste Spezifität und war fähig auch Gemische der beiden Verbindungen in verschiedenen Mengenverhältnissen abzubauen. Um diese nicht adaptierten Mikroorganismenproben zu aktivieren war jedoch eine relativ lange Induktionsperiode nötig. Die drei Proben wurden gemischt und kultiviert, indem der Kolben mit Sauerstoff gesättigt, dann die chlorierten Substrate eingespritzt und nach Abbau der Substrate jeweils erneut Sauerstoff eingeleitet und Substrat eingespritzt wurde. Auf diese Weise wurde in aufeinanderfolgenden Subkulturen eine Anreicherung an leistungsfähigen Bakterien erreicht. Während für den Abbau der Substrate bei den ersten Injektionen mehrere Tage gebraucht wurden, waren nach guter Akklimatisation nur noch einige Stunden nötig.

Bei Injektion von je 112 mg Monochlorbenzol und 22 mg o-Dichlorbenzol pro 1 Kulturmedium verlief der Bioabbau von Injektion zu Injektion immer schneller, was bedeutet, dass das Mikroorganismenkonsortium immer effizienter wurde, die chlorierten Substrate abzubauen. Bei Erhöhung der o-Dichlorbenzolkonzentration von 22 auf 44 mg/l trat jedoch eine Verzögerungsphase auf, und der o-Dichlorbenzolabbau wurde langsamer; überdies ergab sich eine leichte Abnahme in der Monochlorbenzol-Abbaugeschwindigkeit.

Die Zunahme des Salzgehaltes im Kulturmedium wurde durch Leitfähigkeitsmessung verfolgt. Die gemessenen Werte, die der stöchiometrischen Freisetzung von Chloridionen entsprachen, bestätigten den vollständigen Abbau von Monochlorbenzol und o-Dichlorbenzol (Mineralisation zu Kohlendioxid und Produktion von Biomasse).

b) <u>Zusammensetzung des ausgewählten Mikroorganismenkonsortiums</u>

Nach mehreren Subkulturen blieb die bakterielle Zusammensetzung des ausgewählten Konsortiums konstant. Durch Isolierung der Stämme auf einer Agarplatte (Plate Count Agar) und anschliessend Identifikation mit der "Biolog Microplate"-Bestimmungsmethode wurden zwei vorherrschende Typen aerober gram-negativer Bakterien gefunden, nämlich Burkholderia sp. und Pseudomonas sp., wobei von letzteren Pseudomonas putida, Pseudomonas mucidolens, Pseudomonas cepecia und Pseudomonas flava identifiziert werden konnten. Als weitere Bakterien, die ebenfalls zu den Pseudomoadaceae gehören, wurden Stenotrophphomonas sp. und Comamonas sp. isoliert.

Über 90% der Zellen erwiesen sich während der Kultivierungsperiode als lebensfähig, wie auf der Basis selektiver Zellmembranpermeabilität gegen spezifische Fluoreszenzsonden (Live/Dead Baclight Viability Kit L-7007, Molecular Probes, Eugene, USA) festgestellt wurde. Nach mehrmonatiger Lagerung bei +5°C waren noch 60% der Zellen lebensfähig.

c) Charakterisierung des Mikroorganismenkonsortiums

Zur Bestimmung des Inhibitionsgrenzwertes der beiden chlorierten Substrate, d.h. der Grenzkonzentration, ab der das Mikroorganismenwachstum gehemmt wird, wurden verschiedene Anfangskonzentrationen (von 0 bis zur Löslichkeitsgrenze) getestet und der Einfluss auf das Wachstum der Biomasse (durch Trübungsmessung) und den Abbau der Substrate (durch Messung ihrer Konzentration in der Gasphase mittels GC) beobachtet. Für Monochlorbenzol wurde bei Anfangskonzentrationen unter 220 mg/l eine Verzögerungsphase von 5 Stunden beobachtet; bei einer Anfangskonzentration von 333 mg/l betrug die Verzögerungsphase 1 Woche. Bei Anfangskonzentrationen von 411 mg/l und höher war kein Wachstum und kein Substratabbau mehr festzustellen und das Kulturmedium färbte sich schwarz. Der Gehalt an nicht abführbarem organischen Kohlenstoff NPOC (Non Purgeable Organic Carbon oder gelöster organischer Kohlenstoff mit Ausschluss von Kohlendioxid) im Kulturmedium stieg an, und in den HPLC- und GC-Analysen erschien ein neuer, unidentifizierter Peak, der möglicherweise verwandten Substanzen von Chlorcatechin oder Chlorchinonen entsprechen könnte, die zur Bildung toxischer Muconsäuren führen könnten.

Analoge Tests für o-Dichlorbenzol ergaben bei Anfangskonzentration über 22 mg/l eine Verzögerungsphase (5 Stunden bei 52 und 92 mg/l). Bei Anfangskonzentrationen von 131 und 156 mg/l wurde kein Wachstum und kein Substratabbau mehr beobachtet.

Die Toxizität von Monochlorbenzol und o-Dichlorbenzol könnte möglicherweise durch ihren lipophilen Charakter und/ oder die Anreicherung intrazellulärer, toxischer Metaboliten bedingt sein. Die nach Injektion hoher Substratmengen beobachtete Toxizität war jedoch reversibel. Durch Waschen der inhibierten Zellen und Einspritzen einer neuen, aber niedrigeren Substratmenge begann der Substratabbau und das Mikroorganismenwachstum erneut.

Die vorangehenden Versuche zeigen, dass die Fähigkeit des Mikroorganismenkonsortiums, zu wachsen und chlorierte Verbindungen abzubauen, mit deren Anfangskonzentration variiert. Die tolerierbaren Grenzkonzentrationen im Kulturmedium betrugen etwa 330 mg/l für Monochlorbenzol und etwa 120 mg/l für o-Dichlorbenzol. Zudem wurde festgestellt, dass o-Dichlorbenzol langsamer abgebaut wurde als Monochlorbenzol, weshalb für die Massenkultivierung ein Gemisch von Monochlorbenzol und o-Dichlorbenzol im Volumenverhältnis von 5:1 verwendet wurde.

Die spezifische Bioabbaugeschwindigkeit des Mikroorganismenkonsortiums für Monochlorbenzol und o-Dichlorbenzol wurde unter Verwendung eines Gemisches von Monochlorbenzol und o-Dichlorbenzol (Volumenverhältnis 5:1) bei Anfangskonzentrationen von 80 mg/l bzw. 20 mg/l bestimmt. Die spezifischen Abbauraten waren 0,2 g Monochlorbenzol und 0,04 g o-Dichlorbenzol pro 1 g Mikroorganismen (Trockengewicht) pro Stunde. Diese Werte wurden mit dem akklimatisierten Konsortium erhalten. Der Abbau von Monochlorbenzol begann sofort, während für den Abbau von o-Dichlorbenzol eine Verzögerungszeit von mindestens 40 Minuten beobachtet wurde (Anfangskonzentration der Biomasse: 0,37 g Trockengewicht pro l).

d) Bestimmung der Substratmenge pro Injektion und Massenkultivierung

Die optimalen Substratmengen pro Injektion ("Pulsamplituden") wurden durch Testen von vier verschiedenen Substratmengen des Gemischen von Monochlorbenzol und o-Dichlorbenzol (Volumenverhältnis 5:1) in einem Bioreaktor im Labormassstab unter Verwendung einer prozessgekoppelt-offenen (open loop) Kultivierungsmethode bestimmt.

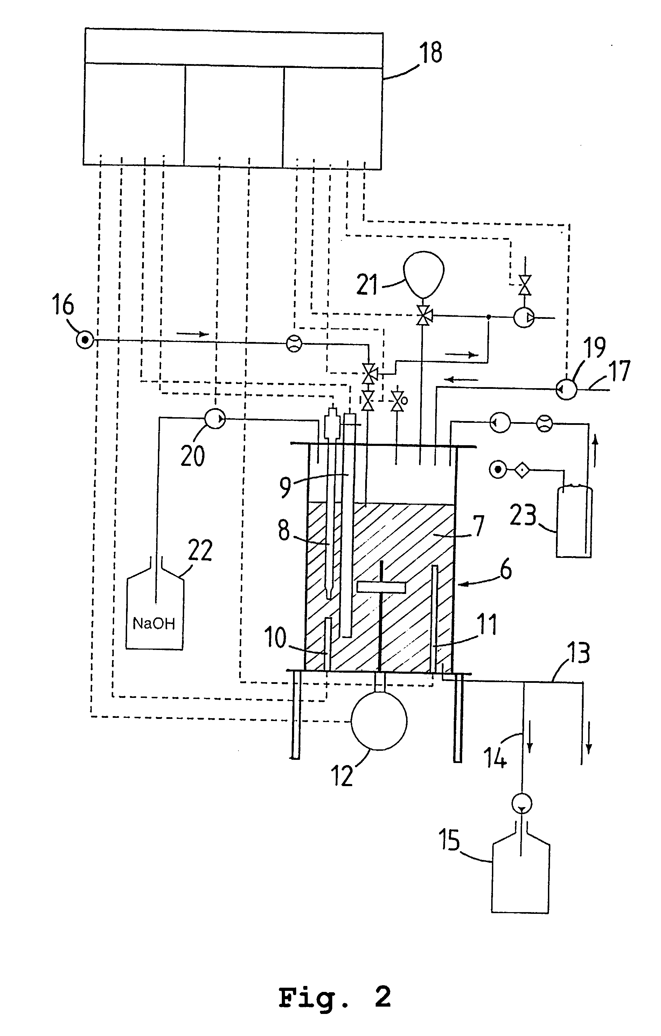

Die Kultivierung des ausgewählten Mikroorganismenkonsortiums erfolgte bei 30°C in einem KFL 2000 Bioreaktor (Bioengineering AG, Wald, Schweiz), der in Fig. 2 zusammen mit den Gas- und Flüssigkeitsleitungen (ausgezogene Linien), den elektrischen Leitungen zur Übertragung der Messund Steuersignale (unterbrochene Linien) und den weiteren Ausrüstungsteilen schematisch dargestellt ist. Die Figur zeigt den eigentlichen Bioreaktor 6 mit flüssigem Kulturmedium 7, einer pH-Sonde 8, einer $pO_2$-Sonde 9 (polarographisch, Messbereich 0-100%), einem Temperaturfühler 10, einer Heizvorrichtung 11, einem Rührer mit Rührmotor 12 (verwendet wurden 3 Rushton-Turbinen bei 900 UpM) und Abflussleitungen 13, 14 zur Entnahme von Proben des Kulturmediums bzw. zum Abernten der Biomasse, die in einem Tank 15 aufgefangen wird, sowie eine Sauerstoffquelle 16, eine Leitung 17 zur Zufuhr des flüssigen Substrates und eine Kontrolleinheit 18, zur Steuerung der Zwei- und Dreiwegventile und der Flüssigkeitspumpen 19, 20 mittels Zeitrelais, um die Belüftung, die Substratmengen und die Substratinjektionsfrequenz automatisch zu steuern. Der KLa-Wert durch Oberflächenbelüftung betrug 100 h$^{-1}$. Um die Sauerstoffverfügbarkeit zu erhöhen und den durch den Sauerstoffverbrauch bedingten Druckabfall zu kompensieren, wurde der Reaktor 6 mit einem 2 1-Tedlar-Ballon 21 (Alltech, Artikel Nr. 41003, Ardsley NY, USA) verbunden. Zur Aufrechterhaltung eines pH-Wertes von 7,0 im Kulturmedium wurde notfalls verdünnte Natronlauge aus einem Vorratsgefäss 22 zudosiert. Die Erneuerung oder Ergänzung des Kulturmediums erfolgte durch Zugabe aus einem Vorratstank 23. Als Kulturmedium wurde eine wässrige Lösung der folgenden Substanzen verwendet:

| Kulturmedium A | |
|---|---|
| $(NH_4)_2SO_4$ | 5 g/l |
| $K_2HPO_4$ | 3,27 g/l |
| $NaH_2PO_4.2H_2O$ | 1,88 g/l |
| $MgSO_4.7H_2O$ | 1 g/l |
| $CaCl_2.2H_2O$ | 50 mg/l |
| $EDTA-2Na.2H_2O$ | 10 mg/l |
| $FeSO_4.7H_2O$ | 1,6 mg/l |
| $ZnSO_4.7H_2O$ | 0,4 mg/l |
| $MnCl_2.4H_2O$ | 0,4 mg/l |
| $NaMO_4.2H_2O$ | 0,4 mg/l |
| $CoCl_2.6H_2O$ | 0,4 mg/l |
| $CuCl_2.2H_2O$ | 0,4 mg/l |
| $H_3BO_3$ | 0,3 mg/l |
| $NiCl_2.6H_2O$ | 0,046 mg/l |
| Biotin | 0,02 mg/l |
| p-Aminobenzoesäure | 0,25 mg/l |
| Nicotinsäure | 0,5 mg/l |
| Thiamin·HCl | 0,5 mg/l |
| Calciumpantothenat | 0,5 mg/l |
| Pyridoxin·HCl | 0,5 mg/l |
| Cyanocobalamin | 0,05 mg/l |
| Riboflavin | 0,2 mg/l |
| Folsäure | 0,1 mg/l |
| Cholin·HCl | 0,5 mg/l |
| Mesoinosit | 2,0 mg/l |

Das ausgewählte Mikroorganismenkonsortium wurdenach Lagerung während 3 Monaten bei +5°C - in einem Bioreaktor auf dem oben definierten Kulturmedium A durch wiederholte, manuelle Zugabe eines Gemisches von 50 ml Monochlorbenzol und 10 ml o-Dichlorbenzol reaktiviert, wobei eine erneute Zugabe jeweils erst erfolgte, wenn das Substrat vollständig verbraucht war (Messung in der Gasphase mittels GC), und vor der Substratzugabe Sauerstoff zugeführt wurde, wenn aufgrund des Sauerstoffprofils keine ausreichende Sauerstoffmenge mehr verfügbar war.

Zur Kultivierung des Mikroorganismenkonsortiums wurde in dem in Fig. 2 gezeigten Bioreaktor 1 1 Kulturmedium A vorgelegt (während des Produktionszyklus erfolgte keine weitere Zugabe an Kulturmedium) und mit dem reaktivierten Mikroorganismenkonsortium angeimpft. Dem System wurde bis zur Sauerstoffsättigung des flüssigen Mediums Sauerstoff zugeführt. Nach der Belüftungsperiode wurde innert kurzer Zeit eine präzise Menge an Substrat (Monochlorbenzol/o-Dichlorbenzol-Gemisch im Volumenverhältnis 5:1) in flüssiger Form in das Kulturmedium eingespritzt. Die Zeit zur Einstellung des Gleichgewichtes zwischen der Gas- und Flüssigphase (nach Henrys Gesetz) wurde für Monochlorbenzol auf 2 Minuten und o-Dichlorbenzol auf 10 Minuten geschätzt. Erneute Belüftung oder Substratzugabe erfolgte erst, wenn das injizierte Substrat vollständig abgebaut war. Wenn aufgrund der Sauerstoffverfügbarkeit im System möglich, wurden vor der nächsten Belüftung mit Sauerstoff mehrere aufeinanderfolgende Substratpulse durchgeführt. Diese Sequenz von Belüften und Substratzugabe wurde wiederholt, bis die höchste, durch die Sauerstofftransferkapazität erlaubte Biomassenkonzentration im Reaktor erreicht war. Dann wurde das Kultivierungsverfahren gestoppt oder ein Teil der Kultur entnommen und durch frisches Kulturmedium ersetzt, um nach der gleichen Methode einen neuen Produktionszyklus zu starten.

Um das Kultivierungsverfahren unter automatischen Bedingungen betreiben zu können, wurden die Belüftung und Substratinjektion mittels Zeitrelais sequentiell geregelt. Die Dauer der Belüftung und der Substratinjektion sowie die Zeit zwischen zwei Injektionen wurden jedoch konstant gehalten, und es wurde keine Closed-loop-Regelung durchgeführt, um die Biomassenproduktivität durch Minimierung der für jedes dieser Ereignisse benötigten Zeit zu erhöhen.

Bei der Kultivierung wurde darauf geachtet, dass die Konzentration an freigesetzten Chloridionen nicht über 0,2 M stieg. Um eine Wachstumsbeschränkung durch Stickstoffmangel zu vermeiden, wurde dem Kulturmedium notfalls Ammoniumsulfat zugesetzt.

e) Ergebnisse

Die Ergebnisse der Kultivierungsverfahren sind in Tabelle 1 zusammengestellt.

Tabelle 1

| Bestimmung der optimalen Pulsamplitude | | | | |
|---|---|---|---|---|
| Substrat pro Injektion: | | | | |
| Monochlorbenzol [ml] o-Dichlorbenzol [ml] | 50 10 | 100 20 | 150 30 | 200 40 |
| Ber. Anfangskonzentration: | | | | |
| Monochlorbenzol [mg/l] o-Dichlorbenzol [mg/l] | 35 10,2 | 70,7 20,4 | 106 30,5 | 141 40,7 |
| Zeit zwischen 2 Injektionen [min.] | 75 | 90 | 90 | 360 |
| Produktionszyklus [h] | 48 | 30 | 53 | 48 |
| Gesamtzahl an Injektionen | 38 | 20 | 35 | 8 |
| volumetrische Ladung an Substrat [kg/m$^3$d] | 1,3 | 2,19 | 3,25 | 1,09 |
| volumetrische Zellenproduktivität [kg*/m$^3$d] | 0,43 | 0,98 | 1,22 | 0 |
| Biomassenausbeute [g*/g Substrat] | 0,33 | 0,45 | 0,38 | 0 |

* Trockengewicht

Für eine "Pulsamplitude" (Substratmenge pro Injektion) von Monochlorbenzol und o-Dichlorbenzol pro Einheitsvolumen der Kultur von weniger als 150 bzw. 30 ml wurde keine Verzögerungszeit für Monochlorbenzol beobachtet, wogegen mehr als 20 Minuten nötig waren, bis eine Abnahme der o-Dichlorbenzol-Konzentration in der Gasphase festgestellt wurde. Der Bioabbau von o-Dichlorbenzol begann erst, als Monochlorbenzol beinahe völlig aufgebraucht war. Tabelle 1 zeigt, dass die Zellenproduktivität mit der volumetrischen Ladung an Substrat anstieg. Da die Zeit zwischen 2 Injektionen konstant gehalten wurde, blieben die Zellen mit steigender Anfangskonzentration an Substrat länger unter maximalen Wachstumsbedingungen. Unter Berücksichtigung der Werte der spezifischen Abbauraten der beiden Substrate kann angenommen werden, dass die maximalen Wachstumsraten des Konsortiums erreicht wurden. Für diese Substratmengen pro Injektion wird die vollständige Mineralisation belegt durch die gute Korrelation zwischen berechneten und gemessenen Konzentrationen an freien Chloridionen. Überdies wurde kein Anstieg an NPOC beobachtet.

Bei Verwendung von 200 ml Monochlorbenzol und 40 ml o-Dichlorbenzol pro Injektion erfolgte eine zunehmende Inhibierung der Zellen. Von Injektion zu Injektion nahm die Abbaurate ab, bis nach der achten Injektion schliesslich kein weiterer Abbau und kein Mikroorganismenwachstum mehr festgestellt wurde. Als der Abbau stoppte, wurden rasche Lyse der Zellen, Klärung des Kulturmediums, Schaumentwicklung und ein Anstieg des pH-Wertes beobachtet. Mittels Doppelstrahl-Spektrophotometer und HPLC wurden neue Peaks entdeckt, die auf Metaboliten (möglicherweise Muconsäurederivate) zurückzuführen sein könnten. Nach 48 Stunden wurde das Kulturmedium schliesslich dunkelbraun und schwarz, und die Zellen hatten eine schwarze Farbe.

f) Schlussfolgerungen

Die maximale volumetrische Zellenproduktivität durch Injektion von Monochlorbenzol und o-Dichlorbenzol betrug 1,22 kg/m$^3$d für eine maximale volumetrische Ladung von 3,25 kg/m$^3$d. Der geschwindigkeitbegrenzende Schritt für den Abbau der chlorierte Substrate war nicht die Elimination der Anfangsverbindungen, sondern der Abbau metabolischer Zwischenprodukte. Bei wiederholten Injektionen trat eine Inhibierung tatsächlich schon ab niedrigeren Grenzkonzentrationen auf (140 mg Monochlorbenzol/l und 40 mg o-Dichlorbenzol/l) als im Falle einer einzelnen Injektion dieser Substrate (330 mg Monochlorbenzol/l und 120 mg o-Dichlorbenzol/l). Um eine Vergiftung der Zellen durch Ansammlung von primären Metaboliten zu vermeiden, wurde die Maximalmenge an Substrat pro Injektion auf 150 ml Monochlorbenzol und 30 ml o-Dichlorbenzol pro Liter Kulturmedium festgelegt. Zur Elimination dieser Zwischenprodukte ist offenbar eine bestimmte Zeit nötig. Dies bestätigt die Ansicht, dass das Mikroorganismenkonsortium mittels "gepulster" Injektionen von Substrat unter einer strengen Kontrolle der injizierten Mengen und der Abbauzeit (d.h. der "Pulsamplituden" Ap und der "Pulsfrequenz" Fp) kultiviert werden muss, und es erklärt, warum es nicht möglich ist, das bakterielle Konsortium durch kontinuierliche Zufuhr von Substrat in die flüssige Phase zu kultivieren. Dieser Befund schliesst a priori die Verwendung von kontinuierlichen oder von "Fed-batch"-Kultivierungstechniken zur Massenproduktion bakterieller Konsortien auf xenobiotischen Verbindungen aus.

Zur einfachen und schnellen Optimierung des obigen Kultivierungsverfahrens unter industriellen Bedingungen und zur Vermeidung der Ansammlung toxischer Metaboliten, hat sich die Absorptionsmessung bei 255 nm als guter Kontroll-parameter erwiesen.

Beispiel 2

Produktion eines spezialisierten Mikroorganismenkonsortiums auf einem Gemisch aus Toluol, Ethylbenzol und o-, m- und p-Xylol unter diskontinuierlicher Belüftung mit Luft

a) Auswahl leistungsfähiger Mikrooganismenkonsortien

In analoger Weise wie in Beispiel 1 wurden in der Abwasserreinigungsanlage der Gemeinde Schweizerhalle (Schweiz) mehrere Mikroorganismenproben aus den kontaminierten Belebtschlämmen und den Abgas-Biofiltrationseinheiten entnommen und in Schüttelkolben auf ihre Fähigkeit zum Abbau eines Gemisches aus 26 Volumenteilen Toluol, 1,5 Volumenteilen Ethylbenzol, 3,7 Volumenteilen m- und p-Xylol und 0,7 Volumenteilen o-Xylol getestet. Während der Akklimatisierungsperiode wurden im Unterschied zu Beispiel 1 jeweils 32 ml des obigen Gemisches als Substrat eingespritzt und erneut die gleiche Menge injiziert, wenn das Substrat völlig aus der Gasphase verschwunden war. Nach mehrmaliger Zugabe wurden durch Verdünnung der Kultur um einen Faktor 10 mit neuem Kulturmedium Subkulturen erstellt. Diese Vorgehensweise führte zu leistungsfähigen, angereicherten Mikroorganismenkonsortien, die sich für die Massenkultivierung eigneten.

b) Zusammensetzung des ausgewählten Konsortiums

Die Isolierung und Identifikation der Stämme des ausgewählten Mikroorganismenkonsortiums erfolgte wie in Beispiel 1, wobei die folgenden dominanten Bakterien gefunden wurden: Pseudomonas mucidolens, Pseudomonas putida Biotyp B, Pseudomonas fluorescens Biotyp C und Comamonas acidovorens. Die weiteren identifizierten Stämme waren: Arthrobacter uratoxydans, Rhodococcus erythropili und Bacillus cereus. Ferner waren zwei unidentifizierte Spezies im Konsortium vorhanden.

c) Charakterisierung des Mikroorganismenkonsortiums

Die toxischen Grenzkonzentrationen wurden in analoger Weise bestimmt wie in Beispiel 1. Als maximal tolerierbare Konzentrationen im Kulturmedium wurden gefunden: 220 mg/l für Toluol (als einzige Verbindung) und 50 mg/l für das Gemisch der $C_8H_{10}$-Isomere (o-, m- und p-Xylol und Ethylbenzol). Ein braunes Medium wurde erhalten in Fällen, wo das Wachstum und der Abbau vollständig inhibiert wurden. Wegen des Unterschieds in der Toxizität von Toluol und der $C_8H_{10}$-Isomere wurde ein Gemisch aus 90 Vol.-% Toluol, 2,5 Vol.-% Ethylbenzol, 6,2 Vol.-% m- und p-Xylol und 1,3 Vol.-% o-Xylol (nachfolgend als "TEX" bezeichnet), für die Massenkultivierung verwendet.

In 500 ml-Schüttelkolben mit 100 ml flüssigem Kulturmedium wurden die spezifische Wachstumsrate und die spezifische Substratabsorptionsrate für Toluol allein, für das Gemisch der $C_8H_{10}$-Isomere allein und für TEX ermittelt. Für Toluol allein war die Anfangskonzentration 200 mg/l; für das Gemisch der $C_8H_{10}$-Isomere allein 20 mg/l; für TEX war die Toluolkonzentration 200 mg/l und die Konzentration an $C_8H_{10}$-Isomeren war 20 mg/l.

Beim Abbau von TEX waren Toluol und Ethylbenzol die ersten Substrate, die ohne Verzögerungsphase verschwanden, während o-Xylol und insbesondere m- und p-Xylol die letzten waren, die nach einer kurzen Verzögerungsphase verschwanden.

Die maximale spezifische Wachstumsrate ($\mu_m$) war 0,12 $h^{-1}$ auf Toluol allein, 0,11 $h^{-1}$ auf dem Gemisch der $C_8H_{10}$-Isomere allein und 0,17 $h^{-1}$ auf TEX. Die entsprechenden maximalen spezifischen Substrataufnahmegeschwindigkeiten (qsm) waren 0,10, 0,054 bzw. 0,11 g Substrat pro g Biomasse (Trockengewicht) pro Stunde. Die Michaelis-Konstante war nicht sehr verschieden auf Toluol allein (1,9 mg/l) und auf TEX (2,6 mg/l), wogegen sie auf dem Gemisch

der $C_8H_{10}$-Isomere unter 1 mg/l lag.

d) <u>Massenkultivierung des ausgewählten Konsortiums auf TEX</u>

Zur Kultivierung des Mikroorganismenkonsortiums durch "gepulste" Injektion von TEX wurde mit Ausnahme der folgenden Unterschiede die gleiche Methode angewendet wie in Beispiel 1 für chlorierte Verbindungen. Die Kultivierung erfolgte in einem voll ausgerüsteten 20 l-CSTR-Bioreaktor (Bioline, Inceltech France, Toulouse) mit ca. 12-13 l flüssigem Kulturmedium und ca. 7-8 l Gasvolumen. Anstelle der Verwendung eines Tedlar-Ballons wurde an der TEX-Zufuhrleitung ein Absperrventil angeordnet und der Reaktor mit der Aussenatmosphäre über eine Kapillare verbunden, um den Verlust an Substrat und Sauerstoff (im Falle der Belüftung mit reinem Sauerstoff) zu begrenzen. Dies erlaubte die Durchführung des Verfahrens unter industrie-ähnlichen Bedingungen. Um die Sauerstofftransfereffizienz des Bioreaktors zu verbessern, wurde überdiese mittels einer Membranpumpe (N026SVE, Haussmann) und einer am Boden des Reaktors angeordnete Verteilereinrichtung kontinuierlich Gas aus der Gasphase in das Kulturmedium rezirkuliert (4,5 l/min.). Auf diese Weise wurde der mit blosser Oberflächenbelüftung erhaltene KLa-Wert von etwa 100 h$^{-1}$ auf mehr als 200 h$^{-1}$ erhöht. Ein visueller Durchflussregler bestätigte die gute Arbeit dieser Pumpe. Vorsichtshalber wurde zwischen dem oberen Teil des Reaktors und der Umwälzpumpe ein 3 l-Tank angeordnet, um im Falle von Schaumentwicklung Gas von Flüssigkeit zu trennen.

Wegen des Stickstoffverbrauchs durch Wachstum heterotropher und nitrifizierender Bakterien (1 g Ammoniumsulfat pro g synthetisierte trockene Biomasse) wurde bei jeder Substratinjektion weiteres Ammoniumsulfat in das Kulturmedium eingebracht, um die Stickstoffentfernung durch das Mikroorganismenkonsortium zu kompensieren. Die Zufuhr erfolgte mit einer peristaltischen Pumpe.

Das Mikroorganismenkonsortium wurde unter nicht sterilen Bedingungen und unter diskontinuierlichem Belüften mit Luft und Injizieren von TEX kultiviert, wobei folgende Zeiten eingehalten wurden: Zeit für die Belüftung ca. 5-7 min., Zeit für einen TEX-Puls ca. 0,5-1 min., Zeit für den Abbau der injizierten TEX-Menge ca. 20-40 min., Gesamtzeit für eine Sequenz (Belüftung, TEX-Puls und TEX-Abbau) ca. 25-48 min. Selbst Bakterien, die während mehr als 3 Monaten im Kühlschrank (+5°C), suspendiert in ihrem Kulturmedium, aufbewahrt wurden, konnten innerhalb von weniger als 2 Tagen durch manuelle Injektionen von 300 g TEX/l leicht reaktiviert werden.

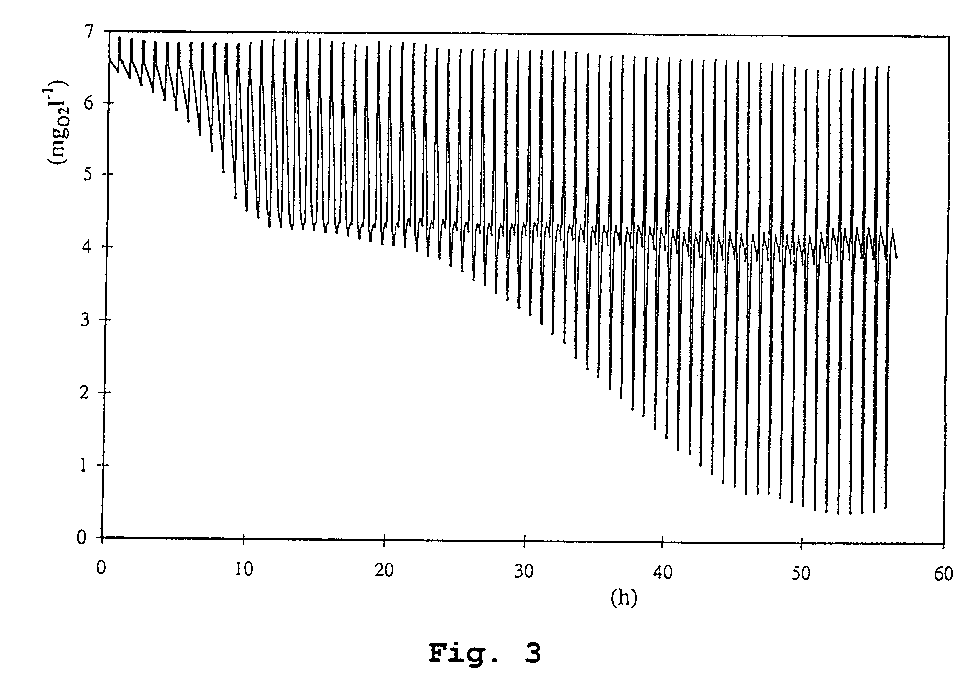

Wegen des sehr raschen Sauerstoffverbrauchs im flüssigen Medium war zwischen zwei Belüftungsperioden jeweils nur eine Injektion von TEX möglich; andernfalls würden sich rasch anoxe Bedingungen etablieren. Nach Zellenreaktivierung wurde tatsächlich rasch das charakteristische V-förmige Profil der gelösten Sauerstoffkonzentration beobachtet; Fig. 3 zeigt eine graphische Darstellung der Konzentration an gelöstem Sauerstoff [mg/l] gegen die Zeit [h]. Dieses Profil lässt sich wie folgt erklären: zuerst, unmittelbar nach der Injektion von TEX, wird Sauerstoff mit einer höheren volumetrischen Respirationsrate verbraucht als die Sauerstofftransferrate des Bioreaktors; wenn TEX (insbesondere Toluol, das am leichtesten abgebaut wird) nahezu völlig aus der Gasphase verschwunden ist, nimmt die Atmung der Zellen ab und die Konzentration an gelöstem Sauerstoff steigt infolge des Sauerstofftransfers aus der Gasphase, bis ein neues Sauerstoffgleichgewicht zwischen der Gas- und Flüssigphase erreicht ist; danach fällt die Konzentration an gelöstem Sauerstoff weiter langsam ab wegen der endogenen Atmung der Zellen und/oder der Oxidation ausgeschiedener metabolischer Zwischenprodukte. Während dieser letzten Phase ist die Sauerstofftransferkapazität des Reaktors hoch genug, um den Sauerstoffverbrauch in der flüssigen Phase nahezu zu kompensieren.

Zur Kultivierung des Mikroorganismenkonsortiums auf TEX wurden nacheinander drei Produktionen, wobei die nächste Kultur jeweils durch Transferieren (und Aufbewahrung im Kühlschrank während einiger Wochen) eines Teils der früheren Kultur erhalten wurde. Bei Beginn der ersten Kultur betrug die Menge an injiziertem TEX 800 mg pro Puls. Nach 300 Stunden Kultivierung zeigten sich indessen Probleme des Sauerstofftransfers. Zu diesem Zeitpunkt betrug die Biomassenkonzentration (Trockengewicht) etwa 3 g/l, und der Sauerstoff wurde rascher verbraucht. Um vorübergehende anoxe Bedingungen zu vermeiden, wurde die pro Puls injizierte Menge TEX auf 400 mg reduziert. Diese Menge wurde auch während der zweiten und dritten Produktion verwendet, weil Sauerstofftransferprobleme schon rasch nach der Inokulation auftraten. Für 400 mg injiziertes TEX betrugen die theoretischen Anfangskonzentrationen von TEX im flüssigen Medium 21,3 mg/l für Toluol, 0,59 mg/l für Ethylbenzol, 1,43 mg/l für m- und p-Xylol und 0,32 mg/l für o-Xylol unter der Annahme, dass Henrys Gleichgewicht praktisch momentan erreicht wird.

Die volumetrischen Ladungen an TEX wurden während der dritten Produktion durch Reduktion der Zeit zwischen aufeinanderfolgenden Injektionen, d.h. durch Steigerung der "Pulsfrequenz", erhöht. Dies illustriert, wie die Biomasse immer effizienter wurde, TEX zu eliminieren.

Die Ergebnisse der Kultivierungsverfahren sind in Tabelle 2 zusammengestellt.

Tabelle 2

| Zellenproduktivität auf TEX unter diskontinuierlicher Belüftung mit Luft | | | |
|---|---|---|---|
| Produktion | 1 | 2 | 3 |
| Gesamtzahl Substratinjektionen | 360 | 760 | 1100 |
| Produktionszyklus [h] | 450 | 378 | 620 |
| Gesamtmenge an injiziertem TEX [g] | 275 | 195 | 600 |
| Anfangskonzentration der Biomasse* [g/l] | 0,25 | 2 | 1,25 |
| Endkonzentration der Biomasse* [g/l] | 5,5 | 8 | 15,7 |
| TEX-Beladungsrate [kg/m$^3$d] | 0,91 | 1,32 | 2,14 |
| Maximale Zellenproduktivität [g*/m$^3$d] | 0,29 | 0,41 | 0,45 |
| Biomassenausbeute [g*/g TEX] | 0,32 | 0,31 | 0,21 |

\* Trockengewicht

Wegen des Wachstums von Biomasse an den Reaktorwänden wird die Zellenproduktivität in obigen Ergebnissen möglicherweise leicht unterschätzt. Während Perioden der Schaumentwicklung war der Spiegel der Flüssigkeit vorübergehend erhöht, was zu Fixierung und Wachstum von Biomasse im oberen Teil des Reaktors führte. Diese Biomasse stellte etwa 10% im Vergleich zu der bei Beendigung der Produktion aus der flüssigen Phase geernteten Biomasse dar.

Anderseits ist zu erwähnen, dass es nicht möglich war, die spezifische Abbaurate von TEX zu bestimmen, weil der Abbau im Vergleich zu der für die GC-Analyse benötigten Zeit (8 Minuten) zu rasch war. Tatsächlich waren 10 Minuten nach Injektion über 90% der eingespritzten Substrate aus der Gasphase entfernt. Toluol und Ethylbenzol zeigten keine Verzögerungsphase und waren innert 10 Minuten verbraucht. o-, m- und p-Xylol zeigten eine Verzögerungsphase von weniger als 10 Minuten, verschwanden aber praktisch vollständig innert 20 Minuten. Von den im Substratgemisch vorhandenen Substanzen schienen m- und p-Xylol am schwierigsten abbaubar; es war jedoch mit den vorhandenen analytischen Mitteln nicht möglich zwischen dem m- und p-Isomer zu unterscheiden.

Während den drei Produktionsverfahren wurde die Bildung von NPOC beobachtet. Die Produktion von NPOC war linear und niedrig während der ersten Produktion, wurde aber zunehmend schneller und höher während den nächsten Produktionen. Es bestand eine gewisse Korrelation zwischen der Produktion von NPOC und der Freisetzung von Proteinen aus den Zellen und parallel zur Zunahme dieser Substanzen verfärbte sich das extrazelluläre Medium immer stärker goldgelb. Die beobachtete Schaumentwicklung war vermutlich auf die Anwesenheit extrazellulärer Proteine zurückzuführen. Am Ende der zweiten und dritten Produktion wurde selbst in Abwesenheit von TEX eine hohe Zellrespiration festgestellt, was möglicherweise der Oxidation intrazellulärer Reserven zugeschrieben werden kann.

Beispiel 3

Produktion eines spezialisierten Mikroorganismenkonsortiums auf einem Gemisch aus Toluol, Ethylbenzol und o-, m- und p-

Xylol unter unter diskontinuierlicher Belüftung mit Sauerstoff

Bei dem in Beispiel 2 durchgeführten Kultivierungsverfahren unter diskontinuierlicher Belüftung mit Luft waren die Zeiten für Belüftung und Substratabbau bereits auf Minimalwerten. Da die Produktion durch den Sauerstofftransfer begrenzt war, wurde im vorliegenden Beispiel untersucht, ob durch Verwendung von Sauerstoff eine Steigerung der volumetrischen Ladung von TEX und dadurch eine Erhöhung der Zellenproduktivität möglich ist.

Belüftung mit reinem Sauerstoff bewirkte eine höhere Sauerstoffverfügbarkeit und eine höhere Sauerstofftransferkapazität. Da die KLa-Werte für Luft und reinen Sauerstoff gleich waren, war die volumetrische Sauerstofftransferrate unter Sättigungsbedingungen tatsächlich proportional zur Sauerstoffkonzentration. Durch Verwendung von Sauerstoff anstelle von Luft stieg dieser Wert auf das 5-fache (die maximale Konzentration an gelöstem Sauerstoff war 7 mg/l bei Verwendung von Luft bzw. 35 mg/l bei Verwendung von reinem Sauerstoff) und die Sauerstofftransferkapazität erhöhte sich ebenfalls. Unter diesen Bedingungen kann grundsätzlich mehr TEX injiziert werden pro Puls.

Das Kultivierungsverfahren auf TEX unter Verwendung von Sauerstoff (nachfolgend als "Produktion 4" bezeichnet) wurde in analoger Weise zu Beispiel 2 und mit dem gleichen Mikroorganismenkonsortium durchgeführt, soweit nicht nachfolgend etwas Abweichendes erwähnt wird.

In Schritt 1 (vgl. Tabelle 3) wurde zur Zellenreaktivierung (nach mehrmonatiger Lagerung in einem Kühlschrank) Luft zu Belüftung verwendet. Die Respirationsrate wuchs von Substratinjektion zu Substratinjektion, wobei wiederum die typische V-Form des Sauerstoffprofils auftrat, wenn TEX vollständig aus der Gasphase verschwunden war. Ab diesem Moment trat endogene Respiration unmittelbar vor der Wiederbelüftung auf. Die Zellenproduktivität war in der gleichen Grössenordnung wie während der ersten drei Produktionen mit Luft (vgl. Beispiel 2).

Nach der Reaktivierung des Mikroorganismenkonsortiums wurde die volumetrische TEX-Ladung erhöht und reiner Sauerstoff zur Belüftung verwendet, um anoxe Bedingungen zu vermeiden. Bei jeder Injektion wurden 3 g TEX eingespritzt. Trotz Verwendung von reinem Sauerstoff wurden wegen der hohen Biomassenkonzentration und der hohen Sauerstoffaufnahmegeschwindigkeit der Zellen anoxe Bedingungen erreicht. Überdies war eine Sauerstoffsättigung des Kulturmedium immer schwerer zu erreichen. Unter diesen Umständen wurden die Ergebnisse als das Beste betrachtet, was mit dieser Verfahrensweise erreicht werden konnte. Es wurde entschieden, jedes Mal, wenn eine hohe Biomassenkonzentration erreicht war, einen Teil der Kultur durch neues Kulturmedium zu ersetzen und einen neuen Produktionszyklus zu starten (Schritte 2 und 3). Am Schluss des zweiten Produktionszyklus (Schritt 4) verlangsamte sich das Zellenwachstum und die Aktivität (Respiration und Abbau). Die Kultur wurde daher verdünnt und mit einer geringeren volumetrischen TEX-Ladung erneut gestartet (Schritt 5). Als die volumetrische TEX-Ladung danach wieder erhöht wurde, war die Biomasse wieder fähig, zu wachsen und TEX mit nahezu der gleichen Effizienz abzubauen wie vorher (Schritt 6).

Tabelle 3

| Produktion 4 des Mikroorganismenkonsortiums auf TEX unter diskontinuierlicher Belüftung mit Sauerstoff | | | | | | |
|---|---|---|---|---|---|---|
| Schritt | 1 | 2 | 3 | 4 | 5 | 6 |
| Anfangskonzentration an Biomasse* [g/l] | 1,0 | 2,4 | 9,4 | 8,6 | 4,0 | 6,8 |
| Endkonzentration an Biomasse* [g/l] | 2,4 | 17,8 | 22,0 | 15,3 | 6,8 | 11,5 |
| TEX-Beladungsrate [ kg/m³d] | 1,2 | 10,8 | 10,9 | 10,0 | 2,1 | 5,0 |
| Maximale Zellenproduktivität [kg*/m³d] | 0,47 | 5,6 | 6,1 | 5,7 | 0,45 | 3,66 |
| Biomassenausbeute [g*/g TEX] | 0,39 | 0,52 | 0,56 | 0,57 | 0,21 | 0,73 |

* Trockengewicht

Im Vergleich mit den Produktionen 1-3 (Beispiel 2) wurden höhere Konzentrationen an NPOC und Proteinen erhalten, und deren Produktionsraten stiegen mit der TEX-Ladung (etwa 0,2 mg NPOC/l.h für Produktionen mit Luft, etwa 1,5 mg NPOC/l.h für Produktionen mit Sauerstoff). Möglicherweise werden die Zellen durch TEX geschädigt, was den Rückgang des Zellwachstums in Produktion 4 erklären könnte. Messungen der Lebensfähigkeit der Zellen, basierend auf der selektiven Membranpermeation von Fluoreszenzsonden, konnte diese Hypothese bestätigen. Tatsächlich waren nicht mehr als 50-70% der Zellen dieser Kultur lebensfähig. Ein weiterer Grund könnte sein, dass die bei jedem Puls injizierte TEX-Menge Anfangskonzentrationen (etwa 160 mg/l für Toluol und etwa 6,2 mg/l für Xylol) entsprach, die nahe an der im Schüttelkolben bestimmten Grenzkonzentration liegt. Da diese Grenzkonzentrationen mit Einzelinjektionen bestimmt wurden, konnten schädliche kumulative Effekte aufeinanderfolgender Injektionen nicht ausgeschlossen werden.

Während des zweiten Schrittes der Produktion 4 wurde TEX für drei aufeinanderfolgende Injektionen durch Xylol ersetzt. Bei 1,3 g Xylol pro Puls wurde der völlige Abbau aller Substrate festgestellt. Im Vergleich zu Toluol war der Abbau der Xylol-Isomeren langsamer (insbesondere im Falle niedriger Restkonzentrationen), aber es wurde keine Verzögerungsphase beobachtet. Die Respirationsrate war ebenfalls langsamer. Dies illustriert die Fähigkeit des Mikroorganismenkonsortiums, TEX-Gemisch verschiedener Mengenverhältnisse effizient abzubauen.

In einer weiteren Produktion (Produktion 5) wurde die pro Puls injizierte TEX-Menge auf 2 g reduziert, um anoxe Bedingungen, wie sie in Produktion 4 auftraten, eher zu vermeiden. Trotzdem wurde die gleiche Situation erreicht. Nach der ersten Verdünnung der Kultur wurde die Zellenaktivität und das Zellwachstum sogar vermindert. Durch einfache Verdünnung mit frischem Kulturmedium wurde das Wachstum und die Aktivität jedoch rasch wiederhergestellt. Die Bildung von NPOC und extrazellulären Proteinen war vergleichbar mit Produktion 4, obwohl die TEX-Ladung niedriger war.

Schlussfolgerungen

Die Zellenkultivierung durch TEX-Pulse erlaubte, hohe Biomassenkonzentrationen in guter Produktivität herzustellen. Da das System während der Abbauperiode nicht belüftet wurde, trat keine Desorption von TEX (Stripping) auf. Da die Zellrespiration jedoch hoch war (und obwohl der Reaktor eine ausgezeichnete Sauerstofftransfereffizienz aufwies), war es nicht möglich, bei Verwendung von Luft oder reinem Sauerstoff mehr als 0,4 g bzw. 2 g TEX in 13 l Kulturmedium zu injizieren. Unter diesen Bedingungen wurden anoxe Bedingungen vermieden. Die maximale Zellenproduktivität war bei Verwendung von Luft 0,5 kg/m$^3$d und bei Verwendung von reinem Sauerstoff 5,0 kg/m$^3$d, bezogen auf das Trockengewicht. Bei niedrigen Zellenproduktivitätsbedingungen wurde eine Abnahme der Zellenaktivität beobachtet. Bei Erhöhung der Zellenproduktivität durch Belüftung mit reinem Sauerstoff und Erhöhung der TEX-Ladung wurde jedoch ebenfalls ein gewisser Verlust an Aktivität beobachtet. Diese Situation könnte möglicherweise durch eine Schädigung der Zellen durch TEX bedingt sein, wie die unter gewissen Umständen niedrige Lebensfähigkeit der Zellen und die Freisetzung von NPOC und extrazellulären Proteinen während der Kultivierung vermuten lassen. Folglich wären die schädigenden Wirkungen möglicherweise abhängig von der zeitlichen Einwirkung von TEX oder injizierten TEX-Menge. Andere Gründe, wie die direkte Inhibition durch Substrate und die Ansammlung toxischer Metaboliten oder Reserven, sind ebenfalls nicht völlig auszuschliessen. Diese letzte Hypothese wurde gestützt durch den Befund, dass hohe Respirationsraten am Ende der Kultivierung in Abwesenheit von Substraten auftraten.

Die erfindungsgemässe Substratpuls-Methode erweist sich somit als eine gut anpassbare Kultivierungsmethode, um durch sehr präzise Regelung der Substratzufuhr auch unter schwierigen Bedingungen Inhibition zu vermeiden und ein zuverlässiges und stabiles Produktionsverfahren zu ermöglichen. Das Signal von der Sonde zur Messung des gelösten Sauerstoffs gibt überdies eine sehr gute und nützliche Information über den Status der Zellen, die dem Operateur notfalls ein rasches Reagieren erlaubt, um die Kultur vor Inhibition zu bewahren. Das vorliegende Beispiel illustriert zudem, dass selbst bei Beeinflussung der Zellenaktivität und des Wachstums durch unkontrollierbare Phänome die Kultivierung durch einfache Verdünnung erneut gestartet werden konnte und die gleiche Leistungsfähigkeit zeigte wie zuvor.

Beispiel 4

Produktion eines spezialisierten Mikroorganismenkonsortiums auf einem Gemisch aus Toluol, Ethylbenzol und o-, m- und p-Xylol unter Belüftung mit Luft

Das in Beispiel 2 erhaltene spezialisierte Mikroorganismenkonsortium wurde in einem CSTR-Bioreaktor mit einem Gesamtvolumen von 1,5 m$^3$ auf TEX kultiviert, wobei im wesentlichen das in Beispiel 2 beschriebene Verfahren im 20 l-Laborbioreaktor massstäblich vergrössert und die Apparatur modifiziert wurde, um einigen industriellen Beschränkungen zu genügen. Die Parameter und Ausrüstung des Bioreaktors und die Verfahrensbedingungen waren wie folgt:

- Gesamtvolumen des Reaktors: 1,5 m$^3$
- Maximales Flüssigkeitsvolumen: 1 m$^3$
- Volumenregelung: durch Überlauf
- Kulturvolumen: 700 l
- Luftdurchsatz (Rotameter): 0-30 m$^3$/h
- Luftdurchsatz für direkte Belüftung: 15 m$^3$/h
- Minimale Belüftungszeit: 6 min.
- Sollkonzentration an gelöstem Sauerstoff für Belüftung: 80%
- Warnvorgabewert für minimale Konzentration an gelöstem Sauerstoff: 1% (während mindestens 2 min.)
- Gasrezirkulationsrate der Membranpumpe: 1,8 m$^3$/h
- KLa durch Oberflächenbelüftung: 19 h$^{-1}$
- KLa (direkte Belüftung): 170 h$^{-1}$
- Rührsystem: 2 Rushton-Turbinen, 1 Propeller im oberen Teil
- Rührgeschwindigkeit: 50-295 UpM (Betrieb bei 295 UpM)
- Injektion von TEX: direkt in die Flüssigphase
- Injiziertes TEX-Volumen pro Puls: 30-35 ml
- Zeit für TEX-Zufuhr: 30-35 s
- Verzögerungszeit (ohne Belüftung): 35-240 s
- Gesamte Abbauzeit (inklusive Verzögerungszeit): 20 min.
- Minimalzeit für einen Zyklus: ca. 26,5 min.
- automatische Sequenzregelung: Siemens Simatic
- Temperaturregelung: 30,0°C
- pH-Regelung (5M NaOH/2M H$_3$PO$_4$): 7,0 + 0,2

Der Bioreaktor wurde diskontinuierlich unter Zugabe von TEX-Pulsen und Belüftung mit Luft betrieben. Wegen der sehr niedrigen Sauerstofftransfereffizienz des Bioreaktors und der hohen Sauerstoffaufnahmegeschwindigkeit der Mikroorganismen, wurde die Belüftung des Reaktors jeweils vor der Injektion von TEX unterbrochen und rasch nach der Injektion von TEX (d.h. noch während der Substratabbauperiode) wieder aufgenommen; andernfalls würden innert einiger Minuten anoxe Bedingungen erreicht. Die restliche Abbauperiode wurde unter konstanter Belüftung durchgeführt, wodurch bis zu einem gewissen Grad Desorption auftreten kann.

Der Luftdurchsatz wurde so gewählt, dass das System rasch belüftet werden konnte, aber nur eine geringe Desorption ("Stripping") und nur ein begrenzter Verlust an Kultur infolge Gasstaus und/oder Schaumentwicklung auftreten konnte. Das Zeitintervall, während dem der Substratabbau ohne Belüftung erfolgte ("Verzögerungszeit"), wurde in Funktion der Amplitude (d.h. der Substratmenge pro Injektion) und der Variationsgeschwindigkeit der Konzentration des gelösten Sauerstoffs variiert. Die anschliessende Abbauzeit unter Belüftung wurde hingegen konstant gehalten, um sicherzugehen, dass vor der nächsten Injektion kein restliches TEX mehr im Reaktor war.

Die Sequenz der wiederholten Substratzufuhr und Belüftung wurde auf einer Zeitbasis geregelt und lässt sich wie folgt zusammenfassen:

- Schritt 1: Belüftung der Kultur während einer minimalen Belüftungszeit, um die Sollkonzentration an gelöstem Sauerstoff (80% der Sättigungskonzentration) zu erreichen;
- Schritt 2: Injektion von TEX direkt in die flüssige Phase (in Abwesenheit von Belüftung);
- Schritt 3: Verzögerungszeit, während der Substratabbau und die Respiration in Abwesenheit von Belüftung erfolgen;
- Schritt 4: Abbauperiode unter Belüftung und Desorption (Stripping);

wobei nach Schritt 4 jeweils die Schritte 2-4 bis zur Beendigung des Produktionszyklus wiederholt wurden.

Verschiedene Warnverfahren (für minimale Sauerstoffkonzentration, pH, Temperatur etc.) sicherten die Kultur im Falle einer Betriebsstörung.

Unter diesen Betriebsbedingungen war die Zellenproduktivität besser als aufgrund der Laborresultate mit Luft als Belüftungsgas erwartet werden konnte, und es wurden hohe Konzentrationen an Biomasse erhalten. Es wurden nacheinander drei Produktionen durchgeführt, wobei die neue Kultur jeweils durch Verdünnen eines Teils der vorangehenden Kultur mit frischem Medium erhalten wurde. Das Verfahren erwies sich als betriebsicher, und es trat kein Aktivitätsverlust auf, d.h. die Kultur war stabil. Dieser Befund könnte darauf zurückzuführen sein, dass die Anfangskonzentration an TEX bei jedem Puls niedrig war. Tatsächlich war die maximale Konzentration im Kulturmedium lediglich 38,7 mg/l für Toluol und 4,3 mg/l für Xylol, unter der Annahme dass die Verbindungen vollständig gelöst in der flüssigen Phase vorliegen. Diese Konzentrationen liegen weit unter den in Beispiel 2 bestimmten Grenzkonzentrationen. Da überdies die Produktivität recht gut war, wurde die Zeit zur Kultivierung reduziert. Ferner war die Biomassenwachstumsausbeute höher als in den Laborversuchen, was möglicherweise der geringeren Bedeutung des Zellenwachstums an der Reaktorwand zugeschrieben werden kann. Die NPOC-Konzentration stieg zwar in den vorliegenden Produktionen während der Kultivierung ebenfalls an, aber es wurde keine Korrelation mit der Zellenaktivität gefunden. Die Desorption (Stripping) war begrenzt, insbesondere bei hohen Biomassenkonzentrationen, wenn die Verzögerungszeit auf 35 s reduziert wurde. Die konstante Schaumentwicklung war das einzige Problem, weil es das nützliche Volumen des Reaktors auf etwa 700 l begrenzte.

Die unter industriellen Bedingungen erhaltene höhere Zellenproduktivität könnte durch den geringeren negativen Beitrag des Zellwachstums an der Wand im Vergleich zum kleinen Labor-Bioreaktor erklärt werden (Tabelle 4).

Tabelle 4

| Zellenproduktivität im Bioreaktor auf TEX unter Belüftung mit Luft | | | |
|---|---|---|---|
| Produktion | 1 | 2 | 3 |
| TEX-Beladungsrate [kg/m³d] | 2,0 | 2,34 | 2,34 |
| Anfangskonzentration an Biomasse* [g/l] | 12,2 | 16,5 | 16,2 |
| Biomassenproduktivität [kg*/m³d] | 1,9 | 3,12 | 2,93 |
| Biomassenausbeute[1] [g*/g TEX] | 0,95 | 1,33 | 1,25 |
| NPOC am Anfang [mg/l] | 126 | 160 | 118 |

* Trockengewicht

[1] Die Biomassenausbeute wurde berechnet unter der Annahme, dass die gesamte TEX-Ladung abgebaut wurde; Zellwachstum an der Wand wurde nicht berücksichtigt.

Tabelle 4   (fortgesetzt)

| Zellenproduktivität im Bioreaktor auf TEX unter Belüftung mit Luft | | | |
|---|---|---|---|
| Produktion | 1 | 2 | 3 |
| NPOC am Schluss [mg/l] | 698 | 440 | 360 |

Ein Teil des produzierten spezialisierten Bakterienkonsortiums wurde für die Biovermehrung des für die Elimination von flüchtigen organischen Verbindungen in industriellen Abgasen entwickelten industriellen Biorieselfilters der Anmelderin verwendet. Etwa 14 kg Trockengewicht an Biomasse wurden chargenweise auf eine mit TEX betriebene 50 m$^3$-Biofiltereinheit über deren Flüssigkeitrezirkulationssystem übertragen. Nach einigen Tagen Berieselung der Biomasse auf der Oberfläche des Bioträgers erhöhte sich die Abbaueffizienz von 60 auf 90%, während die Ladung an Toluol hoch war (2,5 g Toluol pro m$^3$ Rohgas). Diese hohe Effizienz wurde nie vorher erreicht. Dies zeigte, wie leistungsfähig die Biomasse war, um das Biorieselfilter, das einige Monate vorher mit nicht-spezialisiertem Belebtschlamm aus einer industriellen Abwasserbehandlungsanlage angeimpft worden war, in Gang zu halten.

**Patentansprüche**

1.   Verfahren zur aeroben Kultivierung von Mikroorganismen in einem Bioreaktor unter Verwendung eines wässrigen Kulturmediums und eines als Kohlenstoffquelle dienenden Substrates, dadurch gekennzeichnet,

dass dem Bioreaktor Sauerstoff zugeführt und das Substrat durch wiederholte diskontinuierliche Zugabe in jeweils einer Menge zugesetzt wird, die das Wachstum der Mikroorganismen nicht inhibiert und für deren Abbau die im Bioreaktor vorhandene verfügbare Sauerstoffmenge ausreicht, dass die Zufuhr von Sauerstoff während der Zugabe von Substrat unterbrochen ist,
dass die Zugabe von Substrat jeweils wiederholt wird, wenn die vorherzugeführte Substratmenge im wesentlichen verbraucht ist, und
dass dem Bioreaktor vor der erneuten Zugabe einer Substratmenge Sauerstoff zugeführt wird, wenn die im Bioreaktor vorhandene verfügbare Sauerstoffmenge nicht ausreicht, um die Substratmenge zu abzubauen.

2.   Verfahren nach Anspruch 1, dadurch gekennzeichnet, dass die Zugabe von Substrat erfolgt, indem flüssiges Substrat in das Kulturmedium eingespritzt wird.

3.   Verfahren nach Anspruch 1 oder 2, dadurch gekennzeichnet, dass Mikroorganismen kultiviert werden, die auf den Abbau flüchtiger organischer Verbindungen und/oder xenobiotischer Verbindungen spezialisiert sind.

4.   Verfahren nach einem der Ansprüche 1 bis 3, dadurch gekennzeichnet, dass Mikroorganismen kultiviert werden, die auf den Abbau chlorierter bzw. nicht chlorierter, aromatischer oder aliphatischer Lösungsmittel spezialisiert sind.

5.   Verfahren nach einem der Ansprüche 1 bis 4, dadurch gekennzeichnet, dass man als Substrat eine Substanz oder ein Gemisch von Substanzen verwendet, auf deren Abbau die Mikroorganismen spezialisiert sind.

6.   Verfahren nach einem der Ansprüche 1 bis 5, dadurch gekennzeichnet, dass Mikroorganismen kultiviert werden, die auf den Abbau chlorierter, aromatischer oder aliphatischer Lösungsmittel spezialisiert sind, und dass als Substrat ein chloriertes, aromatisches oder aliphatisches Lösungsmittel, auf dessen Abbau die Mikroorganismen spezialisiert sind, oder ein Gemisch solcher Lösungsmittel verwendet wird.

7.   Verfahren nach einem der Ansprüche 1 bis 5, dadurch gekennzeichnet, dass Mikroorganismen kultiviert werden, die auf den Abbau nicht chlorierter, aromatischer oder aliphatischer Lösungsmittel spezialisiert sind, und dass als Substrat ein nicht chloriertes, aromatisches oder aliphatisches Lösungsmittel, auf dessen Abbau die Mikroorganismen spezialisiert sind, oder ein Gemisch solcher Lösungsmittel verwendet wird.

8.   Verfahren nach einem der Ansprüche 1 bis 7, dadurch gekennzeichnet, dass eine gemischte Mikroorganismenpopulation kultiviert wird.

9.   Verfahren nach einem der Ansprüche 1 bis 8, dadurch gekennzeichnet, dass in der Umwelt vorkommende, aus einem mit dem abzubauenden Substrat kontaminierten Milieu isolierte Mikroorganismen kultiviert werden.

10. Verfahren nach einem der Ansprüche 1 bis 9, dadurch gekennzeichnet, dass Mikroorganismen der Gattung Pseudomonas, Nocardia, Alcaligenes, Arthrobacter, Xanthobacter, Comamonas, Rhodococcus, Geobacter, Geotrichum, Azoarcus, Spingomonas, Aeromonas, Corynebacterium und/oder Burkholderia kultiviert werden.

11. Verfahren nach einem der Ansprüche 1 bis 10, dadurch gekennzeichnet, dass das Kulturmedium neben dem diskontinuierlich zugesetzten Substrat keine weitere Kohlenstoffquelle enthält.

12. Verfahren nach einem der Ansprüche 1 bis 11, dadurch gekennzeichnet, dass bei jeder Zugabe eine konstante Menge an Substrat zugesetzt wird.

13. Verfahren nach einem der Ansprüche 1 bis 12, dadurch gekennzeichnet, dass die Zugabe von Substrat jeweils nach gleichbleibenden Zeitintervallen erfolgt.

14. Verfahren nach Anspruch 13, dadurch gekennzeichnet, dass die Zufuhr von Sauerstoff jeweils nach gleichbleibenden Zeitintervallen erfolgt.

15. Verfahren nach einem der Ansprüche 1 bis 12, dadurch gekennzeichnet, dass die Zugabe von Substrat und die Zufuhr von Sauerstoff jeweils nach variablen Zeitintervallen in Abhängigkeit der Konzentration an gelöstem Sauerstoff im flüssigen Kulturmedium erfolgen.

16. Verfahren nach einem der Ansprüche 1 bis 15, gekennzeichnet durch eine Wiederholung der Sequenz von Verfahrensschritten, umfassend die Zufuhr von Sauerstoff, Unterbrechung der Sauerstoffzufuhr und ein- oder mehrmalige Zugabe von Substrat.

17. Verfahren nach einem der Ansprüche 1 bis 16, dadurch gekennzeichnet, dass das Verfahren in einem kontinuierlich gerührten Bioreaktor durchgeführt wird.

18. Verfahren nach einem der Ansprüche 1 bis 17, dadurch gekennzeichnet, dass zur Zufuhr von Sauerstoff reiner Sauerstoff, Luft, mit Sauerstoff angereicherte Luft oder ein Sauerstoff/Stickstoff-Gemisch in den Bioreaktor eingeleitet wird.

19. Verfahren nach einem der Ansprüche 1 bis 18, dadurch gekennzeichnet, dass der Sauerstoff während des Abbaus des Substrates intern rezirkuliert wird.

20. Verfahren nach einem der Ansprüche 1 bis 19, dadurch gekennzeichnet, dass die Kultivierung fortgesetzt wird, bis im wesentlichen die aufgrund der Sauerstofftransferkapazität des Bioreaktors erhältliche Menge an Biomasse gebildet ist, und dann die Biomasse geerntet wird.

Fig. 1

**Fig. 2**

Fig. 3

EP 0 952 212 A1

Europäisches
Patentamt

**EUROPÄISCHER RECHERCHENBERICHT**

Nummer der Anmeldung

EP 99 81 0324

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | US 4 468 455 A (HOPKINS THOMAS R) 28. August 1984 (1984-08-28) * Ansprüche * --- | 1 | C12M1/36 |
| A | US 4 306 026 A (MASLEN FRANK P ET AL) 15. Dezember 1981 (1981-12-15) * Ansprüche * --- | 1 | |
| A | EP 0 345 588 A (BIOTECHNOLOG FORSCHUNG GMBH) 13. Dezember 1989 (1989-12-13) --- | | |
| A | WO 95 13394 A (CYTOKINETIC INC ;APPLIED POWER CONCEPTS INC (US); ANDREWS FRANK T) 18. Mai 1995 (1995-05-18) --- | | |
| A | FR 2 669 935 A (AJINOMOTO KK) 5. Juni 1992 (1992-06-05) * Ansprüche * --- | 1 | |
| A | US 3 926 738 A (NYIRI LASZLO P ET AL) 16. Dezember 1975 (1975-12-16) ----- | | RECHERCHIERTE SACHGEBIETE (Int.Cl.6) C12M |

Der vorliegende Recherchenbericht wurde für alle Patentansprüche erstellt

| Recherchenort | Abschlußdatum der Recherche | Prüfer |
|---|---|---|
| DEN HAAG | 30. Juli 1999 | Coucke, A |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

......................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**            EP 99 81 0324

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-07-1999

| Im Recherchenbericht angeführtes Patentdokument | | Datum der Veröffentlichung | Mitglied(er) der Patentfamilie | | Datum der Veröffentlichung |
|---|---|---|---|---|---|
| US 4468455 | A | 28-08-1984 | KEINE | | |
| US 4306026 | A | 15-12-1981 | GB | 1525535 A | 20-09-1978 |
| | | | AT | 364337 B | 12-10-1981 |
| | | | AT | 234777 A | 15-03-1981 |
| | | | AU | 516883 B | 25-06-1981 |
| | | | AU | 2385977 A | 12-10-1978 |
| | | | BE | 853186 A | 03-10-1977 |
| | | | BR | 7702085 A | 08-02-1978 |
| | | | CA | 1092039 A | 23-12-1980 |
| | | | CH | 637424 A | 29-07-1983 |
| | | | DE | 2714708 A | 13-10-1977 |
| | | | DK | 143477 A,B, | 03-10-1977 |
| | | | FR | 2430975 A | 08-02-1980 |
| | | | FR | 2438683 A | 09-05-1980 |
| | | | IE | 45274 B | 28-07-1982 |
| | | | IN | 156056 A | 04-05-1985 |
| | | | JP | 1259453 C | 12-04-1985 |
| | | | JP | 52122688 A | 15-10-1977 |
| | | | JP | 59034109 B | 20-08-1984 |
| | | | LU | 77063 A | 01-06-1978 |
| | | | NL | 7703573 A | 04-10-1977 |
| | | | PH | 14144 A | 05-03-1981 |
| | | | PT | 66391 A,B | 01-05-1977 |
| | | | SE | 432439 B | 02-04-1984 |
| | | | SE | 7703773 A | 03-10-1977 |
| | | | TR | 19495 A | 01-05-1979 |
| | | | ZA | 7701910 A | 29-03-1978 |
| | | | DD | 132795 A | 01-11-1978 |
| | | | SU | 1331434 A | 15-08-1987 |
| EP 0345588 | A | 13-12-1989 | WO | 8912091 A | 14-12-1989 |
| | | | JP | 3500847 T | 28-02-1991 |
| WO 9513394 | A | 18-05-1995 | US | 5506117 A | 09-04-1996 |
| | | | AU | 1094595 A | 29-05-1995 |
| FR 2669935 | A | 05-06-1992 | JP | 5030985 A | 09-02-1993 |
| | | | JP | 5076346 A | 30-03-1993 |
| | | | BE | 1008008 A | 12-12-1995 |
| | | | CN | 1064890 A,B | 30-09-1992 |
| | | | CN | 1183474 A | 03-06-1998 |
| | | | FR | 2676234 A | 13-11-1992 |
| | | | IT | 1256566 B | 11-12-1995 |
| | | | US | 5912113 A | 15-06-1993 |

EPO FORM P0461

Für nähere Einzelheiten zu diesem Anhang : siehe Amtsblatt des Europäischen Patentamts, Nr. 12/82

**ANHANG ZUM EUROPÄISCHEN RECHERCHENBERICHT**
**ÜBER DIE EUROPÄISCHE PATENTANMELDUNG NR.**    EP 99 81 0324

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-07-1999

| Im Recherchenbericht angeführtes Patentdokument | Datum der Veröffentlichung | Mitglied(er) der Patentfamilie | Datum der Veröffentlichung |
|---|---|---|---|
| US 3926738        A | 16-12-1975 | CH      558017 A | 15-01-1975 |
|  |  | DE     2323416 A | 29-11-1973 |
|  |  | FR     2184035 A | 21-12-1973 |
|  |  | GB     1434613 A | 05-05-1976 |
|  |  | JP    49050989 A | 17-05-1974 |
|  |  | JP    56051760 B | 08-12-1981 |
|  |  | US     3926737 A | 16-12-1975 |

EPO FORM P0461

Für nähere Einzelheiten zu diesem Anhang : siehe Amtsblatt des Europäischen Patentamts, Nr.12/82